# EUROPEAN PATENT APPLICATION

(11) **EP 3 395 860 A1**
(43) Date of publication of application: **31.10.2018**
(21) Application number: 16878809.9
(22) Date of filing: 21.12.2016
(51) Int. Cl.: C08J 3/07, C07K 14/435, C12N 15/09, D01F 4/00

(54) **METHOD FOR PRODUCING POLYMER AGGREGATE**

(30) Priority: 25.12.2015 JP 2015254210
(71) Applicant: Spiber Inc., Yamagata 997-0052 (JP)
(72) Inventor: OHTA Yoshinori, Tsuruoka-shi Yamagata 997-0052 (JP); SUZUMURA Hiroaki, Tsuruoka-shi Yamagata 997-0052 (JP)
(74) Representative: Piotrowicz, Pawel Jan Andrzej
(86) International application number: PCT/JP2016/088205
(87) International publication number: WO 2017/110922

(57) **Abstract**

The present invention relates to a method for producing a polymer aggregate, the method including: a step for bringing a polymer solution containing a polymer substance and cations for gel formation into contact with an anion solution containing anions for gel formation to form a gel at an interface between the polymer solution and the anion solution; and a step for causing the polymer substance to aggregate inside the gel.

SEQUENCE LISTING

<110>  Spiber Inc.
       KOJIMA INDUSTRIES CORPORATION

<120>  A Method for Producing Macromolecule Aggregate

<130>  15PS007WO1

<150>  JP2015-254210
<151>  2015-12-25

<160>  17

<170>  PatentIn version 3.5

<210>  1
<211>  1154
<212>  PRT
<213>  Artificial Sequence

<220>
<223>  ADF3Kai-Large-NRSH1

<400>  1

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala

145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Ser Arg Ala Arg Ala Gly Ser Gly Gln Gln Gly Pro Gly Gln Gln
545                 550                 555                 560

Gly Pro Gly Gln Gln Gly Pro Gly Gln Gln Gly Pro Tyr Gly Pro Gly
                565                 570                 575

Ala Ser Ala Ala Ala Ala Ala Ala Gly Gly Tyr Gly Pro Gly Ser Gly
            580                 585                 590

Gln Gln Gly Pro Ser Gln Gln Gly Pro Gly Gln Gln Gly Pro Gly Gly
        595                 600                 605

Gln Gly Pro Tyr Gly Pro Gly Ala Ser Ala Ala Ala Ala Ala Ala Gly
    610                 615                 620

Gly Tyr Gly Pro Gly Ser Gly Gln Gln Gly Pro Gly Gly Gln Gly Pro
625                 630                 635                 640

Tyr Gly Pro Gly Ser Ser Ala Ala Ala Ala Ala Ala Gly Gly Asn Gly
                645                 650                 655

Pro Gly Ser Gly Gln Gln Gly Ala Gly Gln Gln Gly Pro Gly Gln Gln
            660                 665                 670

Gly Pro Gly Ala Ser Ala Ala Ala Ala Ala Ala Gly Gly Tyr Gly Pro
        675                 680                 685

Gly Ser Gly Gln Gln Gly Pro Gly Gln Gln Gly Pro Gly Gly Gln Gly
    690                 695                 700

Pro Tyr Gly Pro Gly Ala Ser Ala Ala Ala Ala Ala Ala Gly Gly Tyr
705                 710                 715                 720

Gly Pro Gly Ser Gly Gln Gly Pro Gly Gln Gln Gly Pro Gly Gly Gln
                725                 730                 735

Gly Pro Tyr Gly Pro Gly Ala Ser Ala Ala Ala Ala Ala Ala Gly Gly
            740                 745                 750

Tyr Gly Pro Gly Ser Gly Gln Gln Gly Pro Gly Gln Gln Gly Pro Gly
        755                 760                 765

Gln Gln Gly Pro Gly Gly Gln Gly Pro Tyr Gly Pro Gly Ala Ser Ala
    770                 775                 780

Ala Ala Ala Ala Ala Gly Gly Tyr Gly Pro Gly Tyr Gly Gln Gln Gly
785                 790                 795                 800

Pro Gly Gln Gln Gly Pro Gly Gly Gln Gly Pro Tyr Gly Pro Gly Ala
                805                 810                 815

Ser Ala Ala Ser Ala Ala Ser Gly Gly Tyr Gly Pro Gly Ser Gly Gln
            820                 825                 830

Gln Gly Pro Gly Gln Gln Gly Pro Gly Gly Gln Gly Pro Tyr Gly Pro
        835                 840                 845

Gly Ala Ser Ala Ala Ala Ala Ala Ala Gly Gly Tyr Gly Pro Gly Ser
    850                 855                 860

Gly Gln Gln Gly Pro Gly Gln Gln Gly Pro Gly Gln Gln Gly Pro Gly
865                 870                 875                 880

Gln Gln Gly Pro Gly Gly Gln Gly Pro Tyr Gly Pro Gly Ala Ser Ala
                885                 890                 895

Ala Ala Ala Ala Ala Gly Gly Tyr Gly Pro Gly Ser Gly Gln Gln Gly
            900                 905                 910

Pro Gly Gln Gln Gly Pro Gly Gln Gln Gly Pro Gly Gln Gln Gly Pro
        915                 920                 925

Gly Gln Gln Gly Pro Gly Gln Gln Gly Pro Gly Gln Gln Gly Pro Gly
    930                 935                 940

Gln Gln Gly Pro Gly Gln Gln Gly Pro Gly Gly Gln Gly Ala Tyr Gly
945                 950                 955                 960

Pro Gly Ala Ser Ala Ala Ala Gly Ala Ala Gly Gly Tyr Gly Pro Gly
                965                 970                 975

Ser Gly Gln Gln Gly Pro Gly Gln Gln Gly Pro Gly Gln Gln Gly Pro
            980                 985                 990

Gly Gln Gln Gly Pro Gly Gln Gln Gly Pro Gly Gln Gln Gly Pro Gly
        995                 1000                1005

Gln Gln Gly Pro Gly Gln Gln Gly Pro Tyr Gly Pro Gly Ala Ser
    1010                1015                1020

Ala Ala Ala Ala Ala Ala Gly Gly Tyr Gly Pro Gly Ser Gly Gln
    1025                1030                1035

Gln Gly Pro Gly Gln Gln Gly Pro Gly Gln Gln Gly Pro Gly Gly
    1040                1045                1050

Gln Gly Pro Tyr Gly Pro Gly Ala Ala Ser Ala Ala Val Ser Val
    1055                1060                1065

Gly Gly Tyr Gly Pro Gln Ser Ser Ser Val Pro Val Ala Ser Ala
    1070                1075                1080

Val Ala Ser Arg Leu Ser Ser Pro Ala Ala Ser Ser Arg Val Ser
    1085                1090                1095

Ser Ala Val Ser Ser Leu Val Ser Ser Gly Pro Thr Lys His Ala
    1100                1105                1110

Ala Leu Ser Asn Thr Ile Ser Ser Val Val Ser Gln Val Ser Ala
    1115                1120                1125

Ser Asn Pro Gly Leu Ser Gly Cys Asp Val Leu Val Gln Ala Leu
    1130                1135                1140

Leu Glu Val Val Ser Ala Leu Val Ser Ile Leu

1145                1150


<210>  2
<211>  660
<212>  PRT
<213>  Artificial Sequence

<220>
<223>  ADF3Kai

<400>  2

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro

450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Gly Gly Tyr Gly Pro Gln Ser Ser Ser Val Pro Val Ala Ser Ala
545                 550                 555                 560

Val Ala Ser Arg Leu Ser Ser Pro Ala Ala Ser Ser Arg Val Ser Ser
                565                 570                 575

Ala Val Ser Ser Leu Val Ser Ser Gly Pro Thr Lys His Ala Ala Leu
            580                 585                 590

Ser Asn Thr Ile Ser Ser Val Val Ser Gln Val Ser Ala Ser Asn Pro
        595                 600                 605

Gly Leu Ser Gly Cys Asp Val Leu Val Gln Ala Leu Leu Glu Val Val
    610                 615                 620

Ser Ala Leu Val Ser Ile Leu Gly Ser Ser Ser Ile Gly Gln Ile Asn
625                 630                 635                 640

Tyr Gly Ala Ser Ala Gln Tyr Thr Gln Met Val Gly Gln Ser Val Ala
                645                 650                 655

Gln Ala Leu Ala
            660

<210>  3
<211>  1183
<212>  PRT
<213>  Artificial Sequence

<220>
<223>  ADF3Kai-Large

<400>  3
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gly Gln Gly Pro Tyr Gly Pro Gly Ser Ser Ala Ala Ala Ala Ala
           115                 120                 125
   Ala Gly Gly Asn Gly Pro Gly Ser Gly Gln Gln Gly Ala Gly Gln Gln
       130                 135                 140
   Gly Pro Gly Gln Gln Gly Pro Gly Ala Ser Ala Ala Ala Ala Ala Ala
   145                 150                 155                 160
   Gly Gly Tyr Gly Pro Gly Ser Gly Gln Gln Gly Pro Gly Gln Gln Gly
                   165                 170                 175
   Pro Gly Gly Gln Gly Pro Tyr Gly Pro Gly Ala Ser Ala Ala Ala Ala
               180                 185                 190
   Ala Ala Gly Gly Tyr Gly Pro Gly Ser Gly Gln Gly Pro Gly Gln Gln
           195                 200                 205
   Gly Pro Gly Gly Gln Gly Pro Tyr Gly Pro Gly Ala Ser Ala Ala Ala
       210                 215                 220
   Ala Ala Ala Gly Gly Tyr Gly Pro Gly Ser Gly Gln Gln Gly Pro Gly
   225                 230                 235                 240
   Gln Gln Gly Pro Gly Gln Gln Gly Pro Gly Gly Gln Gly Pro Tyr Gly
   245                 250                 255
   Pro Gly Ala Ser Ala Ala Ala Ala Ala Ala Gly Gly Tyr Gly Pro Gly
               260                 265                 270
   Tyr Gly Gln Gln Gly Pro Gly Gln Gln Gly Pro Gly Gly Gln Gly Pro
           275                 280                 285
   Tyr Gly Pro Gly Ala Ser Ala Ala Ser Ala Ala Ser Gly Gly Tyr Gly
       290                 295                 300
   Pro Gly Ser Gly Gln Gln Gly Pro Gly Gln Gln Gly Pro Gly Gly Gln
   305                 310                 315                 320
   Gly Pro Tyr Gly Pro Gly Ala Ser Ala Ala Ala Ala Ala Ala Gly Gly
                   325                 330                 335
   Tyr Gly Pro Gly Ser Gly Gln Gln Gly Pro Gly Gln Gln Gly Pro Gly
               340                 345                 350
   Gln Gln Gly Pro Gly Gln Gln Gly Pro Gly Gly Gln Gly Pro Tyr Gly
           355                 360                 365
   Pro Gly Ala Ser Ala Ala Ala Ala Ala Ala Gly Gly Tyr Gly Pro Gly
       370                 375                 380
   Ser Gly Gln Gln Gly Pro Gly Gln Gln Gly Pro Gly Gln Gln Gly Pro
   385                 390                 395                 400
   Gly Gln Gln Gly Pro Gly Gln Gln Gly Pro Gly Gln Gln Gly Pro Gly
                   405                 410                 415
   Gln Gln Gly Pro Gly Gln Gln Gly Pro Gly Gln Gln Gly Pro Gly Gly
               420                 425                 430
   Gln Gly Ala Tyr Gly Pro Gly Ala Ser Ala Ala Ala Gly Ala Ala Gly
           435                 440                 445
   Gly Tyr Gly Pro Gly Ser Gly Gln Gln Gly Pro Gly Gln Gln Gly Pro
       450                 455                 460
   Gly Gln Gln Gly Pro Gly Gln Gln Gly Pro Gly Gln Gln Gly Pro Gly
   465                 470                 475                 480
   Gln Gln Gly Pro Gly Gln Gln Gly Pro Gly Gln Gln Gly Pro Tyr Gly
                   485                 490                 495
   Pro Gly Ala Ser Ala Ala Ala Ala Ala Ala Gly Gly Tyr Gly Pro Gly
               500                 505                 510
   Ser Gly Gln Gln Gly Pro Gly Gln Gln Gly Pro Gly Gln Gln Gly Pro
           515                 520                 525
   Gly Gly Gln Gly Pro Tyr Gly Pro Gly Ala Ala Ser Ala Ala Val Ser
       530                 535                 540
   Val Ser Arg Ala Arg Ala Gly Ser Gly Gln Gln Gly Pro Gly Gln Gln
   545                 550                 555                 560
   Gly Pro Gly Gln Gln Gly Pro Gly Gln Gln Gly Pro Tyr Gly Pro Gly
                   565                 570                 575
   Ala Ser Ala Ala Ala Ala Ala Ala Gly Gly Tyr Gly Pro Gly Ser Gly
               580                 585                 590
   Gln Gln Gly Pro Ser Gln Gln Gly Pro Gly Gln Gln Gly Pro Gly Gly
           595                 600                 605
   Gln Gly Pro Tyr Gly Pro Gly Ala Ser Ala Ala Ala Ala Ala Ala Gly
       610                 615                 620
   Gly Tyr Gly Pro Gly Ser Gly Gln Gln Gly Pro Gly Gly Gln Gly Pro
   625                 630                 635                 640
   Tyr Gly Pro Gly Ser Ser Ala Ala Ala Ala Ala Ala Gly Gly Asn Gly
                   645                 650                 655
   Pro Gly Ser Gly Gln Gln Gly Ala Gly Gln Gln Gly Pro Gly Gln Gln
               660                 665                 670
   Gly Pro Gly Ala Ser Ala Ala Ala Ala Ala Ala Gly Gly Tyr Gly Pro
           675                 680                 685
   Gly Ser Gly Gln Gln Gly Pro Gly Gln Gln Gly Pro Gly Gly Gln Gly
       690                 695                 700
   Pro Tyr Gly Pro Gly Ala Ser Ala Ala Ala Ala Ala Ala Gly Gly Tyr
   705                 710                 715                 720
   Gly Pro Gly Ser Gly Gln Gly Pro Gly Gln Gln Gly Pro Gly Gly Gln
                   725                 730                 735
   Gly Pro Tyr Gly Pro Gly Ala Ser Ala Ala Ala Ala Ala Ala Gly Gly
               740                 745                 750
   Tyr Gly Pro Gly Ser Gly Gln Gln Gly Pro Gly Gln Gln Gly Pro Gly
           755                 760                 765
   Gln Gln Gly Pro Gly Gly Gln Gly Pro Tyr Gly Pro Gly Ala Ser Ala
       770                 775                 780
   Ala Ala Ala Ala Ala Gly Gly Tyr Gly Pro Gly Tyr Gly Gln Gln Gly
   785                 790                 795                 800
   Pro Gly Gln Gln Gly Pro Gly Gly Gln Gly Pro Tyr Gly Pro Gly Ala
                   805                 810                 815
   Ser Ala Ala Ser Ala Ala Ser Gly Gly Tyr Gly Pro Gly Ser Gly Gln
               820                 825                 830
   Gln Gly Pro Gly Gln Gln Gly Pro Gly Gly Gln Gly Pro Tyr Gly Pro
           835                 840                 845
   Gly Ala Ser Ala Ala Ala Ala Ala Ala Gly Gly Tyr Gly Pro Gly Ser
       850                 855                 860
   Gly Gln Gln Gly Pro Gly Gln Gln Gly Pro Gly Gln Gln Gly Pro Gly
   865                 870                 875                 880
   Gln Gln Gly Pro Gly Gly Gln Gly Pro Tyr Gly Pro Gly Ala Ser Ala
                   885                 890                 895
   Ala Ala Ala Ala Ala Gly Gly Tyr Gly Pro Gly Ser Gly Gln Gln Gly
               900                 905                 910
   Pro Gly Gln Gln Gly Pro Gly Gln Gln Gly Pro Gly Gln Gln Gly Pro
           915                 920                 925
   Gly Gln Gln Gly Pro Gly Gln Gln Gly Pro Gly Gln Gln Gly Pro Gly
       930                 935                 940
   Gln Gln Gly Pro Gly Gln Gln Gly Pro Gly Gly Gln Gly Ala Tyr Gly
   945                 950                 955                 960
   Pro Gly Ala Ser Ala Ala Ala Gly Ala Ala Gly Gly Tyr Gly Pro Gly
                   965                 970                 975
   Ser Gly Gln Gln Gly Pro Gly Gln Gln Gly Pro Gly Gln Gln Gly Pro
               980                 985                 990
   Gly Gln Gln Gly Pro Gly Gln Gln Gly Pro Gly Gln Gln Gly Pro Gly
           995                 1000                1005
   Gln Gln Gly Pro Gly Gln Gln Gly Pro Tyr Gly Pro Gly Ala Ser
       1010                    1015                    1020
   Ala Ala Ala Ala Ala Ala Gly Gly Tyr Gly Pro Gly Ser Gly Gln
       1025                    1030                    1035
   Gln Gly Pro Gly Gln Gln Gly Pro Gly Gln Gln Gly Pro Gly Gly
       1040                    1045                    1050
   Gln Gly Pro Tyr Gly Pro Gly Ala Ala Ser Ala Ala Val Ser Val
       1055                    1060                    1065
   Gly Gly Tyr Gly Pro Gln Ser Ser Ser Val Pro Val Ala Ser Ala
       1070                    1075                    1080
   Val Ala Ser Arg Leu Ser Ser Pro Ala Ala Ser Ser Arg Val Ser
       1085                    1090                    1095
   Ser Ala Val Ser Ser Leu Val Ser Ser Gly Pro Thr Lys His Ala
       1100                    1105                    1110
   Ala Leu Ser Asn Thr Ile Ser Ser Val Val Ser Gln Val Ser Ala
       1115                    1120                    1125
   Ser Asn Pro Gly Leu Ser Gly Cys Asp Val Leu Val Gln Ala Leu
       1130                    1135                    1140
   Leu Glu Val Val Ser Ala Leu Val Ser Ile Leu Gly Ser Ser Ser
       1145                    1150                    1155
   Ile Gly Gln Ile Asn Tyr Gly Ala Ser Ala Gln Tyr Thr Gln Met
       1160                    1165                    1170
   Val Gly Gln Ser Val Ala Gln Ala Leu Ala
       1175                    1180

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5
<211>  1983
<212>  DNA
<213>  Artificial Sequence

<220>
<223>  ADF3Kai

<400>  5
atgcatcacc atcatcatca tcaccaccac cattcctcgg gctcatcctt ggaagtgtta      60

tttcaaggac cagcacgagc cggttcggga caacaagggc ctggccagca gggcccaggt     120

caacaagggc caggacagca gggtccttat gggcccggcg caagcgcagc agctgcggcc     180

gctggtggct atggtcctgg ctccggtcaa cagggccctt cgcaacaagg tcccgggcag     240

caaggtcctg gtggccaggg tccctacggg ccgggggcga gtgcggcagc agccgctgca     300

ggcggttatg gtccaggaag cggacagcaa ggtccgggag gtcaaggtcc gtatggccca     360

ggctctagcg cggctgccgc tgccgcgggt ggcaacggac cagggagcgg acaacagggc     420

gcgggacaac agggtccagg acagcaaggc ccaggggcgt cggcggctgc agcggcggcc     480

ggaggctatg gacccggctc aggacaacag ggaccgggtc aacaaggacc cggtggccaa     540

ggcccctatg gcccgggcgc cagcgcggcc gcagccgccg cgggcgggta cggccccggt     600

agcggccagg gaccaggtca gcaggggcca ggaggtcagg gcccatacgg tccgggcgca     660

tccgcggcgg cggcagcggc aggtggctac ggtcccggaa gcggccaaca ggggccaggg     720

caacaaggac caggacaaca aggtcctggg ggccaaggac cgtatggacc aggagcatca     780

gctgcagccg cggcagctgg cggttacggt ccaggctacg gccagcaggg tccgggtcag     840

cagggaccgg gaggccaggg gccttatggc cctggcgctt ccgcagccag tgccgcttct     900

ggaggatacg ggccgggaag cggtcagcaa ggccctggcc aacaaggacc tggaggccaa     960

gggccctacg gcccaggagc ctcggcagcc gcagctgccg caggtgggta tgggccaggt    1020

agcgggcaac aagggccggg tcagcaagga ccggggcaac agggacctgg gcagcaagga    1080

cccgggggtc aaggcccgta cggacctggt gcgtctgcag ctgctgctgc ggctggtgga    1140

tatggtccgg gatcggggca gcagggtccc ggtcagcagg gccctggtca gcaagggcca    1200

ggccaacagg gacccggaca acaaggcccg ggtcaacagg gtcctggaca gcaggggccg    1260

ggccaacaag gccctgggca acagggtccg gggggacagg gggcctatgg gcctggcgca    1320

tctgccgccg ctggcgcagc cggtgggtac gggcctgggt caggtcaaca ggggcctggt    1380

caacaaggcc ccgggcaaca gggccccggc cagcaaggtc cagggcagca gggcccggga    1440

cagcaagggc ctggacaaca ggggcccgga cagcagggac cttacgggcc cggtgcgagc    1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcagtgt ccgttggagg ttacggccct cagtcttcgt ctgttccggt ggcgtccgca 1680

gttgcgagta gactgtcttc acctgctgct tcatcgcgag tatcgagcgc tgtttcgtct 1740

cttgtctcgt cgggtcccac gaaacatgcc gccctttcaa atacgatttc atctgtagtg 1800

tcccaagtta gtgcaagtaa cccggggtta tccggatgcg acgttctcgt tcaggcactc 1860

ctagaagtag tatccgcgtt ggtgagcatc ttaggcagct cctcgatagg tcaaataaac 1920

tatggtgctt cagcccagta tacacagatg gtgggacaga gcgtcgcgca ggcattggct 1980

taa 1983

<210> 6
<211> 3552
<212> DNA
<213> Artificial Sequence

<220>
<223> ADF3Kai-Large

<400> 6
atgcatcacc atcatcatca tcaccaccac cattcctcgg gctcatcctt ggaagtgtta 60

tttcaaggac cagcacgagc cggttcggga caacaagggc ctggccagca gggcccaggt 120

caacaagggc caggacagca gggtccttat gggcccggcg caagcgcagc agctgcggcc 180

gctggtggct atggtcctgg ctccggtcaa cagggccctt cgcaacaagg tcccgggcag 240

caaggtcctg gtggccaggg tccctacggg ccgggggcga gtgcggcagc agccgctgca 300

ggcggttatg gtccaggaag cggacagcaa ggtccgggag gtcaaggtcc gtatggccca 360

ggctctagcg cggctgccgc tgccgcgggt ggcaacggac cagggagcgg acaacagggc 420

gcgggacaac agggtccagg acagcaaggc ccaggggcgt cggcggctgc agcggcggcc 480

ggaggctatg gacccggctc aggacaacag ggaccgggtc aacaaggacc cggtggccaa 540

ggcccctatg gcccgggcgc cagcgcggcc gcagccgccg cgggcgggta cggccccggt 600

agcggccagg gaccaggtca gcaggggcca ggaggtcagg gcccatacgg tccgggcgca 660

tccgcggcgg cggcagcggc aggtggctac ggtcccggaa gcggccaaca ggggccaggg 720

caacaaggac caggacaaca aggtcctggg ggccaaggac cgtatggacc aggagcatca 780

gctgcagccg cggcagctgg cggttacggt ccaggctacg gccagcaggg tccgggtcag 840

cagggaccgg gaggccaggg gccttatggc cctggcgctt ccgcagccag tgccgcttct 900

ggaggatacg ggccgggaag cggtcagcaa ggccctggcc aacaaggacc tggaggccaa 960

gggccctacg gcccaggagc ctcggcagcc gcagctgccg caggtgggta tgggccaggt 1020

agcgggcaac aagggccggg tcagcaagga ccggggcaac agggacctgg gcagcaagga 1080

cccgggggtc aaggcccgta cggacctggt gcgtctgcag ctgctgctgc ggctggtgga 1140

tatggtccgg gatcggggca gcagggtccc ggtcagcagg gccctggtca gcaagggcca 1200

ggccaacagg gacccggaca acaaggcccg ggtcaacagg gtcctggaca gcaggggccg 1260

ggccaacaag gccctgggca acagggtccg gggggacagg gggcctatgg gcctggcgca 1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcagtgt ccgtttctag agcacgagcc ggttcgggac aacaagggcc tggccagcag 1680

ggcccaggtc aacaagggcc aggacagcag ggtccttatg ggcccggcgc aagcgcagca 1740

gctgcggccg ctggtggcta tggtcctggc tccggtcaac agggcccttc gcaacaaggt 1800

cccgggcagc aaggtcctgg tggccagggt ccctacgggc cgggggcgag tgcggcagca 1860

gccgctgcag gcggttatgg tccaggaagc ggacagcaag gtccgggagg tcaaggtccg 1920

tatggcccag gctctagcgc ggctgccgct gccgcgggtg gcaacggacc agggagcgga 1980

caacagggcg cgggacaaca gggtccagga cagcaaggcc caggggcgtc ggcggctgca 2040

gcggcggccg gaggctatgg acccggctca ggacaacagg gaccgggtca acaaggaccc 2100

ggtggccaag gcccctatgg cccgggcgcc agcgcggccg cagccgccgc gggcgggtac 2160

ggccccggta gcggccaggg accaggtcag caggggccag gaggtcaggg cccatacggt 2220

ccgggcgcat ccgcggcggc ggcagcggca ggtggctacg gtcccggaag cggccaacag 2280

gggccagggc aacaaggacc aggacaacaa ggtcctgggg gccaaggacc gtatggacca 2340

ggagcatcag ctgcagccgc ggcagctggc ggttacggtc caggctacgg ccagcagggt 2400

ccgggtcagc agggaccggg aggccagggg ccttatggcc ctggcgcttc cgcagccagt 2460

gccgcttctg gaggatacgg gccgggaagc ggtcagcaag gccctggcca acaaggacct 2520

ggaggccaag ggccctacgg cccaggagcc tcggcagccg cagctgccgc aggtgggtat 2580

gggccaggta gcgggcaaca agggccgggt cagcaaggac cggggcaaca gggacctggg 2640

cagcaaggac ccgggggtca aggcccgtac ggacctggtg cgtctgcagc tgctgctgcg 2700

gctggtggat atggtccggg atcggggcag cagggtcccg gtcagcaggg ccctggtcag 2760

caagggccag gccaacaggg acccggacaa caaggcccgg gtcaacaggg tcctggacag 2820

caggggccgg gccaacaagg ccctgggcaa cagggtccgg ggggacaggg ggcctatggg 2880

cctggcgcat ctgccgccgc tggcgcagcc ggtgggtacg ggcctgggtc aggtcaacag 2940

gggcctggtc aacaaggccc cgggcaacag ggccccggcc agcaaggtcc agggcagcag 3000

ggcccgggac agcaagggcc tggacaacag gggcccggac agcagggacc ttacgggccc 3060

ggtgcgagcg cagcggccgc cgccgcaggg ggatatggcc ccggatcggg ccagcaggga 3120

ccaggccagc aaggacctgg ccaacagggc ccggggggtc aggggccgta tggtcccggc 3180

gctgcaagtg ctgcagtgtc cgttggaggt tacggccctc agtcttcgtc tgttccggtg 3240

gcgtccgcag ttgcgagtag actgtcttca cctgctgctt catcgcgagt atcgagcgct 3300

gtttcgtctc ttgtctcgtc gggtcccacg aaacatgccg ccctttcaaa tacgatttca 3360

tctgtagtgt cccaagttag tgcaagtaac ccggggttat ccggatgcga cgttctcgtt 3420

caggcactcc tagaagtagt atccgcgttg gtgagcatct taggcagctc ctcgataggt 3480

caaataaact atggtgcttc agcccagtat acacagatgg tgggacagag cgtcgcgcag 3540

gcattggctt aa 3552

<210> 7
<211> 3465
<212> DNA
<213> Artificial Sequence

<220>
<223> ADF3Kai-Large-NRSH1

<400> 7
atgcatcacc atcatcatca tcaccaccac cattcctcgg gctcatcctt ggaagtgtta 60

tttcaaggac cagcacgagc cggttcggga caacaagggc ctggccagca gggcccaggt 120

caacaagggc caggacagca gggtccttat gggcccggcg caagcgcagc agctgcggcc 180

gctggtggct atggtcctgg ctccggtcaa cagggccctt cgcaacaagg tcccgggcag 240

caaggtcctg gtggccaggg tccctacggg ccgggggcga gtgcggcagc agccgctgca 300

ggcggttatg gtccaggaag cggacagcaa ggtccgggag gtcaaggtcc gtatggccca 360

ggctctagcg cggctgccgc tgccgcgggt ggcaacggac cagggagcgg acaacagggc 420

gcgggacaac agggtccagg acagcaaggc ccaggggcgt cggcggctgc agcggcggcc 480

ggaggctatg gacccggctc aggacaacag ggaccgggtc aacaaggacc cggtggccaa 540

ggcccctatg gcccgggcgc cagcgcggcc gcagccgccg cgggcgggta cggccccggt 600

agcggccagg gaccaggtca gcaggggcca ggaggtcagg gcccatacgg tccgggcgca 660

tccgcggcgg cggcagcggc aggtggctac ggtcccggaa gcggccaaca ggggccaggg 720

caacaaggac caggacaaca aggtcctggg ggccaaggac cgtatggacc aggagcatca 780

gctgcagccg cggcagctgg cggttacggt ccaggctacg gccagcaggg tccgggtcag 840

cagggaccgg gaggccaggg gccttatggc cctggcgctt ccgcagccag tgccgcttct 900

ggaggatacg ggccgggaag cggtcagcaa ggccctggcc aacaaggacc tggaggccaa 960

gggccctacg gcccaggagc ctcggcagcc gcagctgccg caggtgggta tgggccaggt 1020

agcgggcaac aagggccggg tcagcaagga ccggggcaac agggacctgg gcagcaagga 1080

cccgggggtc aaggcccgta cggacctggt gcgtctgcag ctgctgctgc ggctggtgga 1140

tatggtccgg gatcggggca gcagggtccc ggtcagcagg gccctggtca gcaagggcca 1200

ggccaacagg gacccggaca acaaggcccg ggtcaacagg gtcctggaca gcaggggccg 1260

ggccaacaag gccctgggca acagggtccg gggggacagg gggcctatgg gcctggcgca 1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcagtgt ccgtttctag agcacgagcc ggttcgggac aacaagggcc tggccagcag 1680

ggcccaggtc aacaagggcc aggacagcag ggtccttatg ggcccggcgc aagcgcagca 1740

gctgcggccg ctggtggcta tggtcctggc tccggtcaac agggcccttc gcaacaaggt 1800

cccgggcagc aaggtcctgg tggccagggt ccctacgggc cgggggcgag tgcggcagca 1860

gccgctgcag gcggttatgg tccaggaagc ggacagcaag gtccgggagg tcaaggtccg 1920

tatggcccag gctctagcgc ggctgccgct gccgcgggtg gcaacggacc agggagcgga 1980

caacagggcg cgggacaaca gggtccagga cagcaaggcc caggggcgtc ggcggctgca 2040

gcggcggccg gaggctatgg acccggctca ggacaacagg gaccgggtca acaaggaccc 2100

ggtggccaag gcccctatgg cccgggcgcc agcgcggccg cagccgccgc gggcgggtac 2160

ggccccggta gcggccaggg accaggtcag caggggccag gaggtcaggg cccatacggt 2220

ccgggcgcat ccgcggcggc ggcagcggca ggtggctacg gtcccggaag cggccaacag 2280

gggccagggc aacaaggacc aggacaacaa ggtcctgggg gccaaggacc gtatggacca 2340

ggagcatcag ctgcagccgc ggcagctggc ggttacggtc caggctacgg ccagcagggt 2400

ccgggtcagc agggaccggg aggccagggg ccttatggcc ctggcgcttc cgcagccagt 2460

gccgcttctg gaggatacgg gccgggaagc ggtcagcaag gccctggcca acaaggacct 2520

ggaggccaag ggccctacgg cccaggagcc tcggcagccg cagctgccgc aggtgggtat 2580

gggccaggta gcgggcaaca agggccgggt cagcaaggac cggggcaaca gggacctggg 2640

cagcaaggac ccgggggtca aggcccgtac ggacctggtg cgtctgcagc tgctgctgcg 2700

gctggtggat atggtccggg atcggggcag cagggtcccg gtcagcaggg ccctggtcag 2760

caagggccag gccaacaggg acccggacaa caaggcccgg gtcaacaggg tcctggacag 2820

caggggccgg gccaacaagg ccctgggcaa cagggtccgg ggggacaggg ggcctatggg 2880

cctggcgcat ctgccgccgc tggcgcagcc ggtgggtacg ggcctgggtc aggtcaacag 2940

gggcctggtc aacaaggccc cgggcaacag ggccccggcc agcaaggtcc agggcagcag 3000

ggcccgggac agcaagggcc tggacaacag gggcccggac agcagggacc ttacgggccc 3060

ggtgcgagcg cagcggccgc cgccgcaggg ggatatggcc ccggatcggg ccagcaggga 3120

ccaggccagc aaggacctgg ccaacagggc ccggggggtc aggggccgta tggtcccggc 3180

gctgcaagtg ctgcagtgtc cgttggaggt tacggccctc agtcttcgtc tgttccggtg 3240

gcgtccgcag ttgcgagtag actgtcttca cctgctgctt catcgcgagt atcgagcgct 3300

gtttcgtctc ttgtctcgtc gggtcccacg aaacatgccg ccctttcaaa tacgatttca 3360

tctgtagtgt cccaagttag tgcaagtaac ccggggttat ccggatgcga cgttctcgtt 3420

caggcactcc tagaagtagt atccgcgttg gtgagcatct tataa 3465

<210> 8
<211> 601
<212> PRT
<213> Artificial Sequence

<220>
<223> PRT410

<400> 8
   Met His His His His His His Ser Ser Gly Ser Ser Gly Pro Gly Gln
   1 5 10 15
   Gln Gly Pro Tyr Gly Pro Gly Ala Ser Ala Ala Ala Ala Ala Gly Gln
   20 25 30
   Asn Gly Pro Gly Ser Gly Gln Gln Gly Pro Gly Gln Ser Gly Gln Tyr
   35 40 45
   Gly Pro Gly Gln Gln Gly Pro Gly Gln Gln Gly Pro Gly Ser Ser Ala
   50 55 60
   Ala Ala Ala Ala Gly Pro Gly Gln Tyr Gly Pro Gly Gln Gln Gly Pro
   65 70 75 80
   Ser Ala Ser Ala Ala Ala Ala Ala Gly Pro Gly Ser Gly Gln Gln Gly
   85 90 95
   Pro Gly Ala Ser Gly Gln Tyr Gly Pro Gly Gln Gln Gly Pro Gly Gln
   100 105 110
   Gln Gly Pro Gly Ser Ser Ala Ala Ala Ala Ala Gly Gln Tyr Gly Ser
   115 120 125
   Gly Pro Gly Gln Gln Gly Pro Tyr Gly Ser Ala Ala Ala Ala Ala Gly
   130 135 140
   Pro Gly Ser Gly Gln Tyr Gly Gln Gly Pro Tyr Gly Pro Gly Ala Ser
   145 150 155 160
   Gly Pro Gly Gln Tyr Gly Pro Gly Gln Gln Gly Pro Ser Ala Ser Ala
   165 170 175
   Ala Ala Ala Ala Gly Ser Gly Gln Gln Gly Pro Gly Gln Tyr Gly Pro
   180 185 190
   Tyr Ala Ser Ala Ala Ala Ala Ala Gly Gln Tyr Gly Ser Gly Pro Gly
   195 200 205
   Gln Gln Gly Pro Tyr Gly Pro Gly Gln Ser Gly Ser Gly Gln Gln Gly
   210 215 220
   Pro Gly Gln Gln Gly Pro Tyr Ala Ser Ala Ala Ala Ala Ala Gly Pro
   225 230 235 240
   Gly Gln Gln Gly Pro Tyr Gly Pro Gly Ser Ser Ala Ala Ala Ala Ala
   245 250 255
   Gly Gln Tyr Gly Tyr Gly Pro Gly Gln Gln Gly Pro Tyr Gly Pro Gly
   260 265 270
   Ala Ser Gly Gln Asn Gly Pro Gly Ser Gly Gln Tyr Gly Pro Gly Gln
   275 280 285
   Gln Gly Pro Gly Gln Ser Ala Ala Ala Ala Ala Gly Pro Gly Gln Gln
   290 295 300
   Gly Pro Tyr Gly Pro Gly Ala Ser Ala Ala Ala Ala Ala Gly Gln Tyr
   305 310 315 320
   Gly Pro Gly Gln Gln Gly Pro Gly Gln Tyr Gly Pro Gly Ser Ser Gly
   325 330 335
   Pro Gly Gln Gln Gly Pro Tyr Gly Pro Gly Ser Ser Ala Ala Ala Ala
   340 345 350
   Ala Gly Gln Tyr Gly Pro Gly Gln Gln Gly Pro Tyr Gly Pro Gly Gln
   355 360 365
   Ser Ala Ala Ala Ala Ala Gly Gln Tyr Gln Gln Gly Pro Gly Gln Gln
       370                 375                 380
   Gly Pro Tyr Gly Pro Gly Ala Ser Gly Pro Gly Gln Gln Gly Pro Tyr
   385                 390                 395                 400
   Gly Pro Gly Ala Ser Ala Ala Ala Ala Ala Gly Pro Gly Gln Tyr Gly
                   405                 410                 415
   Pro Gly Gln Gln Gly Pro Ser Ala Ser Ala Ala Ala Ala Ala Gly Gln
               420                 425                 430
   Tyr Gly Ser Gly Pro Gly Gln Tyr Gly Pro Tyr Gly Pro Gly Gln Ser
           435                 440                 445
   Gly Pro Gly Ser Gly Gln Gln Gly Gln Gly Pro Tyr Gly Pro Gly Ala
       450                 455                 460
   Ser Ala Ala Ala Ala Ala Gly Gln Tyr Gly Pro Gly Gln Gln Gly Pro
   465                 470                 475                 480
   Tyr Gly Pro Gly Gln Ser Ala Ala Ala Ala Ala Gly Pro Gly Ser Gly
                   485                 490                 495
   Gln Tyr Gly Pro Gly Ala Ser Gly Gln Asn Gly Pro Gly Ser Gly Gln
               500                 505                 510
   Tyr Gly Pro Gly Gln Gln Gly Pro Gly Gln Ser Ala Ala Ala Ala Ala
           515                 520                 525
   Gly Gln Tyr Gln Gln Gly Pro Gly Gln Gln Gly Pro Tyr Gly Pro Gly
       530                 535                 540
   Ala Ser Ala Ala Ala Ala Ala Gly Gln Tyr Gly Ser Gly Pro Gly Gln
   545                 550                 555                 560
   Gln Gly Pro Tyr Gly Pro Gly Gln Ser Gly Ser Gly Gln Gln Gly Pro
                   565                 570                 575
   Gly Gln Gln Gly Pro Tyr Ala Ser Ala Ala Ala Ala Ala Gly Pro Gly
               580                 585                 590
   Ser Gly Gln Gln Gly Pro Gly Ala Ser
           595                 600

<210>  9
<211>  559
<212>  PRT

<213>  Artificial Sequence

<220>
<223>  Recombinant spider silk protein Flag_92_short2

<400>  9

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro
    130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  10
<211>  252
<212>  PRT
<213>  Artificial Sequence

<220>
<223>  Collagen-type4-Kai

<400>  10

Met His His His His His His Ser Ser Gly Ser Ser Lys Asp Gly Val
1               5                   10                  15

Pro Gly Phe Pro Gly Ser Glu Gly Val Lys Gly Asn Arg Gly Phe Pro
            20                  25                  30

Gly Leu Met Gly Glu Asp Gly Ile Lys Gly Gln Lys Gly Asp Ile Gly
        35                  40                  45

Pro Pro Gly Phe Arg Gly Pro Thr Glu Tyr Tyr Asp Thr Tyr Gln Glu
    50                  55                  60

Lys Gly Asp Glu Gly Thr Pro Gly Pro Pro Gly Pro Arg Gly Ala Arg
65                  70                  75                  80

Gly Pro Gln Gly Pro Ser Gly Pro Pro Gly Val Pro Gly Ser Pro Gly
                85                  90                  95

Ser Ser Arg Pro Gly Leu Arg Gly Ala Pro Gly Trp Pro Gly Leu Lys
            100                 105                 110

Gly Ser Lys Gly Glu Arg Gly Arg Pro Gly Lys Asp Ala Met Gly Thr
        115                 120                 125

Pro Gly Ser Pro Gly Cys Ala Gly Ser Pro Gly Leu Pro Gly Ser Pro
    130                 135                 140

Gly Pro Pro Gly Pro Pro Gly Asp Ile Val Phe Arg Lys Gly Pro Pro
145                 150                 155                 160

Gly Asp His Gly Leu Pro Gly Tyr Leu Gly Ser Pro Gly Ile Pro Gly
                165                 170                 175

Val Asp Gly Pro Lys Gly Glu Pro Gly Leu Leu Cys Thr Gln Cys Pro
            180                 185                 190

Tyr Ile Pro Gly Pro Pro Gly Leu Pro Gly Leu Pro Gly Leu His Gly
        195                 200                 205

Val Lys Gly Ile Pro Gly Arg Gln Gly Ala Ala Gly Leu Lys Gly Ser
    210                 215                 220

Pro Gly Ser Pro Gly Asn Thr Gly Leu Pro Gly Phe Pro Gly Phe Pro
225                 230                 235                 240

Gly Ala Gln Gly Asp Pro Gly Leu Lys Gly Glu Lys
                245                 250

<210>  11
<211>  310
<212>  PRT
<213>  Artificial Sequence

<220>
<223>  Resilin-Kai

<400>  11

Met His His His His His His Pro Glu Pro Pro Val Asn Ser Tyr Leu
1               5                   10                  15

Pro Pro Ser Asp Ser Tyr Gly Ala Pro Gly Gln Ser Gly Pro Gly Gly
            20                  25                  30

Arg Pro Ser Asp Ser Tyr Gly Ala Pro Gly Gly Gly Asn Gly Gly Arg
        35                  40                  45

Pro Ser Asp Ser Tyr Gly Ala Pro Gly Gln Gly Gln Gly Gln Gly Gln
    50                  55                  60

Gly Gln Gly Gly Tyr Ala Gly Lys Pro Ser Asp Ser Tyr Gly Ala Pro
65                  70                  75                  80

Gly Gly Gly Asp Gly Asn Gly Gly Arg Pro Ser Ser Ser Tyr Gly Ala

                85                  90                  95

Pro Gly Gly Gly Asn Gly Gly Arg Pro Ser Asp Thr Tyr Gly Ala Pro
            100                 105                 110

Gly Gly Gly Asn Gly Gly Arg Pro Ser Asp Thr Tyr Gly Ala Pro Gly
        115                 120                 125

Gly Gly Gly Asn Gly Asn Gly Gly Arg Pro Ser Ser Ser Tyr Gly Ala
    130                 135                 140

Pro Gly Gln Gly Gln Gly Asn Gly Asn Gly Gly Arg Pro Ser Ser Ser
145                 150                 155                 160

Tyr Gly Ala Pro Gly Gly Gly Asn Gly Gly Arg Pro Ser Asp Thr Tyr
                165                 170                 175

Gly Ala Pro Gly Gly Gly Asn Gly Gly Arg Pro Ser Asp Thr Tyr Gly
            180                 185                 190

Ala Pro Gly Gly Gly Asn Asn Gly Gly Arg Pro Ser Ser Ser Tyr Gly
        195                 200                 205

Ala Pro Gly Gly Gly Asn Gly Gly Arg Pro Ser Asp Thr Tyr Gly Ala
    210                 215                 220

Pro Gly Gly Gly Asn Gly Asn Gly Ser Gly Gly Arg Pro Ser Ser Ser
225                 230                 235                 240

Tyr Gly Ala Pro Gly Gln Gly Gln Gly Gly Phe Gly Gly Arg Pro Ser
                245                 250                 255

Asp Ser Tyr Gly Ala Pro Gly Gln Asn Gln Lys Pro Ser Asp Ser Tyr
            260                 265                 270

Gly Ala Pro Gly Ser Gly Asn Gly Asn Gly Gly Arg Pro Ser Ser Ser
        275                 280                 285

Tyr Gly Ala Pro Gly Ser Gly Pro Gly Gly Arg Pro Ser Asp Ser Tyr
    290                 295                 300

Gly Pro Pro Ala Ser Gly
305                 310

<210> 12
<211> 282
<212> PRT
<213> Artificial Sequence

<220>
<223>  elastin short

<400>  12

Met His His His His His His Ser Ser Gly Ser Ser Leu Gly Val Ser
1               5                   10                  15

Ala Gly Ala Val Val Pro Gln Pro Gly Ala Gly Val Lys Pro Gly Lys
            20                  25                  30

Val Pro Gly Val Gly Leu Pro Gly Val Tyr Pro Gly Gly Val Leu Pro
        35                  40                  45

Gly Ala Arg Phe Pro Gly Val Gly Val Leu Pro Gly Val Pro Thr Gly
    50                  55                  60

Ala Gly Val Lys Pro Lys Ala Pro Gly Val Gly Gly Ala Phe Ala Gly
65                  70                  75                  80

Ile Pro Gly Val Gly Pro Phe Gly Gly Pro Gln Pro Gly Val Pro Leu
                85                  90                  95

Gly Tyr Pro Ile Lys Ala Pro Lys Leu Pro Gly Gly Tyr Gly Leu Pro
            100                 105                 110

Tyr Thr Thr Gly Lys Leu Pro Tyr Gly Tyr Gly Pro Gly Gly Val Ala
        115                 120                 125

Gly Ala Ala Gly Lys Ala Gly Tyr Pro Thr Gly Thr Gly Val Gly Pro
    130                 135                 140

Gln Ala Ala Ala Ala Ala Ala Ala Lys Ala Ala Ala Lys Phe Gly Ala
145                 150                 155                 160

Gly Ala Ala Gly Val Leu Pro Gly Val Gly Gly Ala Gly Val Pro Gly
                165                 170                 175

Val Pro Gly Ala Ile Pro Gly Ile Gly Gly Ile Ala Gly Val Gly Thr
            180                 185                 190

Pro Ala Ala Ala Ala Ala Ala Ala Ala Ala Ala Lys Ala Ala Lys Tyr
        195                 200                 205

Gly Ala Ala Ala Gly Leu Val Pro Gly Gly Pro Gly Phe Gly Pro Gly
    210                 215                 220

Val Val Gly Val Pro Gly Ala Gly Val Pro Gly Val Gly Val Pro Gly

225                 230                 235                 240

Ala Gly Ile Pro Val Val Pro Gly Ala Gly Ile Pro Gly Ala Ala Val
                245                 250                 255

Pro Gly Val Val Ser Pro Glu Ala Ala Ala Lys Ala Ala Ala Lys Ala
            260                 265                 270

Ala Lys Tyr Gly Ala Arg Pro Gly Val Gly
        275                 280

<210>  13
<211>  468
<212>  PRT
<213>  Artificial Sequence

<220>
<223>  type I keratin 26

<400>  13

Met Ser Phe Arg Leu Ser Gly Val Ser Arg Arg Leu Cys Ser Gln Ala
1               5                   10                  15

Gly Thr Gly Arg Leu Thr Gly Gly Arg Thr Gly Phe Arg Ala Gly Asn
            20                  25                  30

Val Cys Ser Gly Leu Gly Ala Gly Ser Ser Phe Ser Gly Pro Leu Gly
        35                  40                  45

Ser Val Ser Ser Lys Gly Ser Phe Ser His Gly Gly Gly Gly Leu Gly
    50                  55                  60

Ser Gly Val Cys Thr Gly Phe Leu Glu Asn Glu His Gly Leu Leu Pro
65                  70                  75                  80

Gly Asn Glu Lys Val Thr Leu Gln Asn Leu Asn Asp Arg Leu Ala Ser
                85                  90                  95

Tyr Leu Asp His Val Cys Thr Leu Glu Glu Ala Asn Ala Asp Leu Glu
            100                 105                 110

Gln Lys Ile Lys Gly Trp Tyr Glu Lys Tyr Gly Pro Gly Ser Gly Arg
        115                 120                 125

Gln Leu Ala His Asp Tyr Ser Lys Tyr Phe Ser Val Thr Glu Asp Leu
    130                 135                 140

Lys Arg Gln Ile Ile Ser Val Thr Thr Cys Asn Ala Ser Ile Val Leu
145                 150                 155                 160

Gln Asn Glu Asn Ala Arg Leu Thr Ala Asp Asp Phe Arg Leu Lys Cys
                165                 170                 175

Glu Asn Glu Leu Ala Leu His Gln Ser Val Glu Ala Asp Ile Asn Gly
            180                 185                 190

Leu His Arg Val Met Asp Glu Leu Thr Leu Cys Thr Ser Asp Leu Glu
        195                 200                 205

Met Gln Cys Glu Ala Leu Ser Glu Glu Leu Thr Tyr Leu Lys Lys Asn
    210                 215                 220

His Gln Glu Glu Met Lys Val Met Gln Gly Ala Ala Arg Gly Asn Val
225                 230                 235                 240

Asn Val Glu Ile Asn Ala Ala Pro Gly Val Asp Leu Thr Val Leu Leu
                245                 250                 255

Asn Asn Met Arg Ala Glu Tyr Glu Asp Leu Ala Glu Gln Asn His Glu
            260                 265                 270

Asp Ala Glu Ala Trp Phe Ser Glu Lys Ser Thr Ser Leu His Gln Gln
        275                 280                 285

Ile Ser Asp Asp Ala Gly Ala Ala Met Ala Ala Arg Asn Glu Leu Met
    290                 295                 300

Glu Leu Lys Arg Asn Leu Gln Thr Leu Glu Ile Glu Leu Gln Ser Leu
305                 310                 315                 320

Leu Ala Met Lys His Ser Tyr Glu Cys Ser Leu Ala Glu Thr Glu Ser
                325                 330                 335

Asn Tyr Cys His Gln Leu Gln Gln Ile Gln Glu Gln Ile Gly Ala Met
            340                 345                 350

Glu Asp Gln Leu Gln Gln Ile Arg Met Glu Thr Glu Gly Gln Lys Leu
        355                 360                 365

Glu His Glu Arg Leu Leu Asp Val Lys Ile Phe Leu Glu Lys Glu Ile
    370                 375                 380

Glu Met Tyr Cys Lys Leu Ile Asp Gly Glu Gly Arg Lys Ser Lys Ser
385                 390                 395                 400

Thr Cys Tyr Lys Ser Glu Gly Arg Gly Pro Lys Asn Ser Glu Asn Gln

405                 410                 415

Val Lys Asp Ser Lys Glu Glu Ala Val Val Lys Thr Val Val Gly Glu
            420                 425                 430

Leu Asp Gln Leu Gly Ser Val Leu Ser Leu Arg Val His Ser Val Glu
        435                 440                 445

Glu Lys Ser Ser Lys Ile Ser Asn Ile Thr Met Glu Gln Arg Leu Pro
    450                 455                 460

Ser Lys Val Pro
465

<210>  14
<211>  20
<212>  DNA
<213>  Artificial Sequence

<220>
<223>  T7 promoter primer

<400>  14
taatacgact cactataggg                                                   20

<210>  15
<211>  27
<212>  DNA
<213>  Artificial Sequence

<220>
<223>  Rep Xba I primer

<400>  15
tctagaaacg gacactgcag cacttgc                                           27

<210>  16
<211>  28
<212>  DNA
<213>  Artificial Sequence

<220>
<223>  Xba I Rep primer

<400>  16
tctagagcac gagccggttc gggacaac                                          28

<210>  17
<211>  19
<212>  DNA
<213>  Artificial Sequence

<220>
<223>  T7 terminator primer

<400> 17
gctagttatt gctcagcgg 19

## Description

### Technical Field

The present invention relates to a method for producing a polymer aggregate.

### Background Art

Spiders' threads are also referred to as spider silks and known to be produced by biosynthetic technologies using natural spider silks as a starting material. Polymer aggregates obtained by molding spider silk proteins in various forms are known. For example, Patent Literature 1 discloses a film using a spider silk protein. In addition, Patent Literature 2 discloses polypeptide particles derived from a spider silk protein.

### Citation List

### Patent Literature

Patent Literature 1: Japanese Unexamined Patent Publication No. 2008-507260
Patent Literature 2: WO2014/61043

### Summary of Invention

### Problems to be Solved by the Invention

However, a protein is easily dissolved in water or other solvents, and even when the protein is extruded in a predetermined form to a coagulation liquid, the protein is dissolved in the coagulation liquid, and thus it is difficult to obtain a molded article in a fibrous form, a film form, or the like in high yield.

In this regard, an object of the present invention is to provide a method for producing a polymer aggregate by which polymer aggregates in various forms can be easily obtained.

### Means for Solving the Problems

The present invention provides a method for producing a polymer aggregate, the method including: a step for bringing a polymer solution containing a polymer substance and cations for gel formation into contact with an anion solution containing anions for gel formation to form a gel at an interface between the polymer solution and the anion solution; and a step for causing the polymer substance to aggregate inside the gel.

In the production method of the present invention, the gel is formed at the interface between the polymer solution and the anion solution by the cations for gel formation and the anions for gel formation. According to this, a solution containing the polymer substance is enclosed inside the formed gel. At this time, the gel functions as a dialysis membrane. Small molecules in the polymer solution and the anion solution (for example, a solvent, a salt, and the like) move through the gel, whereas the polymer substance remains inside the gel and aggregates to form a polymer aggregate. In the production method, the form of the polymer aggregate thus obtained depends on the form of the gel thus formed. Since the gel is formed by contact between the polymer solution and the anion solution, the gel can be formed in various forms such as a fiber, a film, a hollow pipe, and a bead, by simple operation. Thus, according to the production method of the present invention, polymer aggregates in various forms can be easily obtained.

The cations for gel formation may be at least one selected from multivalent cations or hydrogen ions. In addition, the anions for gel formation may be alginate ions.

The anion solution preferably further contains an aggregation promoter for the polymer substance. According to this, a polymer aggregate can be produced in a shorter time.

The polymer solution may further contain a dissolution promoter for the polymer substance.

In the production method of the present invention, since the polymer substance does not move through the gel, a polymer aggregate can be produced in a water system. Thus, solvents of the polymer solution and the anion solution may be water. Since the polymer aggregate can be produced in the water system, processes such as waste liquid treatment can be simplified, and there is no risk that non-aqueous solvent is mixed into the polymer aggregate thus formed. Thus, there is also an advantage that application of the polymer aggregate can be easily widened.

The production method of the present invention is based on the action that the gel functions as a dialysis membrane and the polymer substance is aggregated inside the gel, and thus the type of the polymer substance is not particularly limited. However, typically, the polymer substance is preferably a protein. In addition, the type of the protein is not particularly limited, but for example, may be a polypeptide derived from a spider silk protein.

### Effects of the Invention

According to the present invention, it is possible to provide a method for producing a polymer aggregate by which polymer aggregates in various forms can be easily obtained. In the production method of the present invention, the form of the polymer aggregate thus obtained depends on the form of the gel thus obtained. Since the gel is formed by contact between the polymer solution and the anion solution, the gel can be formed in various forms such as a fiber, a film, a hollow pipe, and a bead, by simple operation.

### Brief Description of Drawings

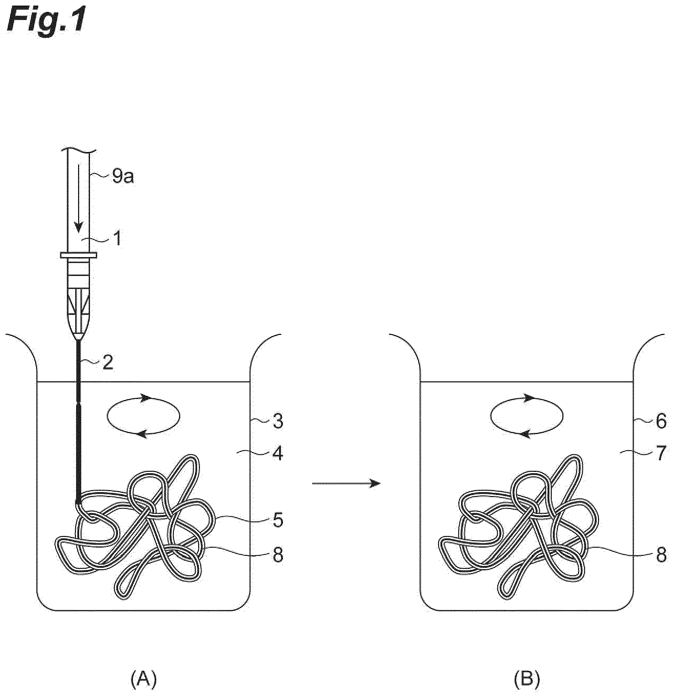

FIG. 1 is an explanatory diagram illustrating a production method according to an embodiment. FIG. 1(A) is an explanatory diagram illustrating a step for extruding a polymer solution (dope) into an anion solution (coagulation liquid). FIG. 1(B) is an explanatory diagram illustrating a step for immersing a polymer aggregate covered with a gel in water and further causing the polymer aggregate to aggregate.

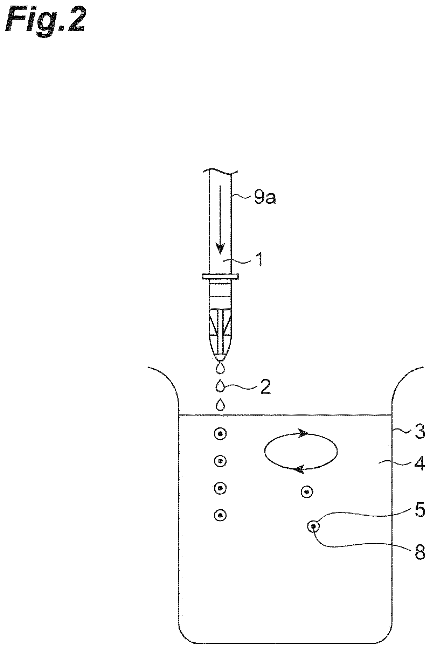

FIG. 2 is an explanatory diagram illustrating a production method according to an embodiment.

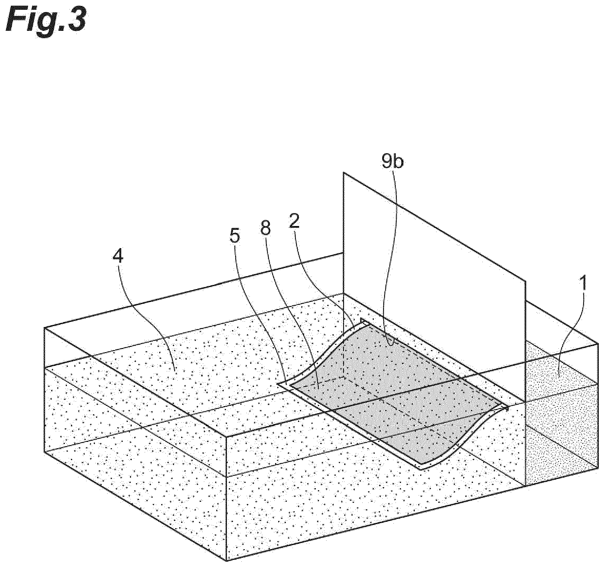

FIG. 3 is an explanatory diagram illustrating a production method according to an embodiment.

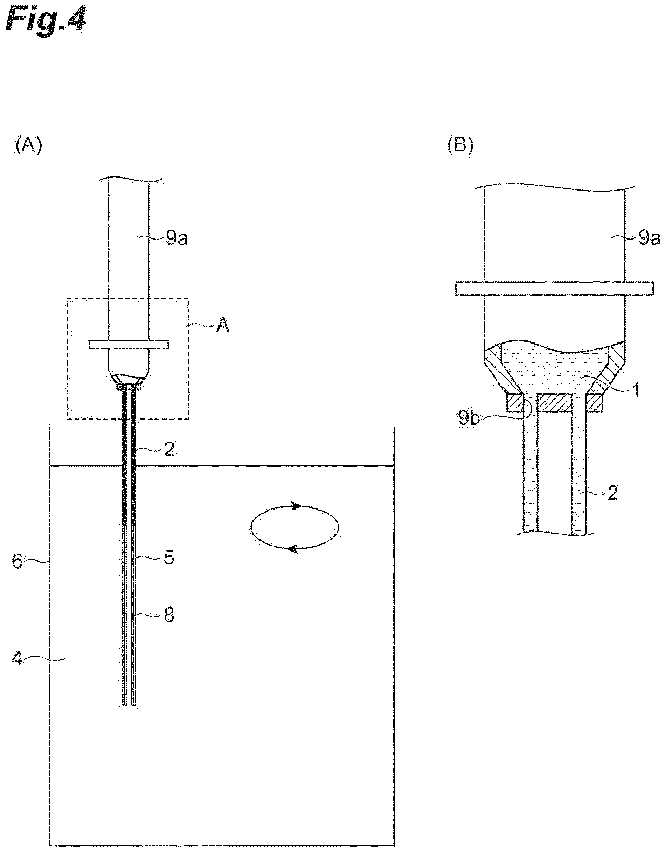

FIG. 4(A) is an explanatory diagram illustrating a production method according to an embodiment. FIG. 4(B) is an enlarged view of a part surrounded by A in FIG. 4(A).

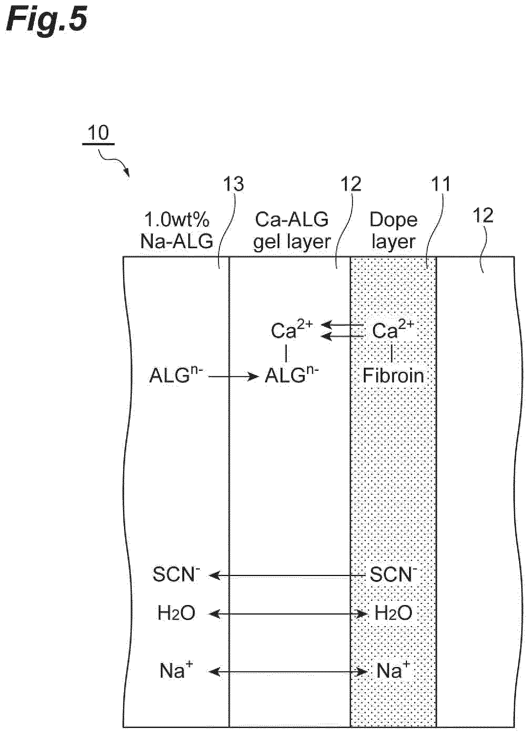

FIG. 5 is a schematic cross-sectional view illustrating a coagulation liquid-gel-dope interface in a production method according to an embodiment.

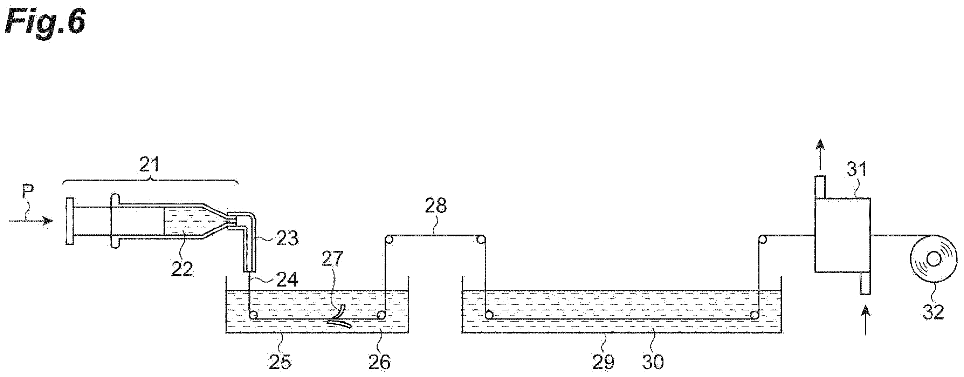

FIG. 6 is a production process diagram according to an embodiment.

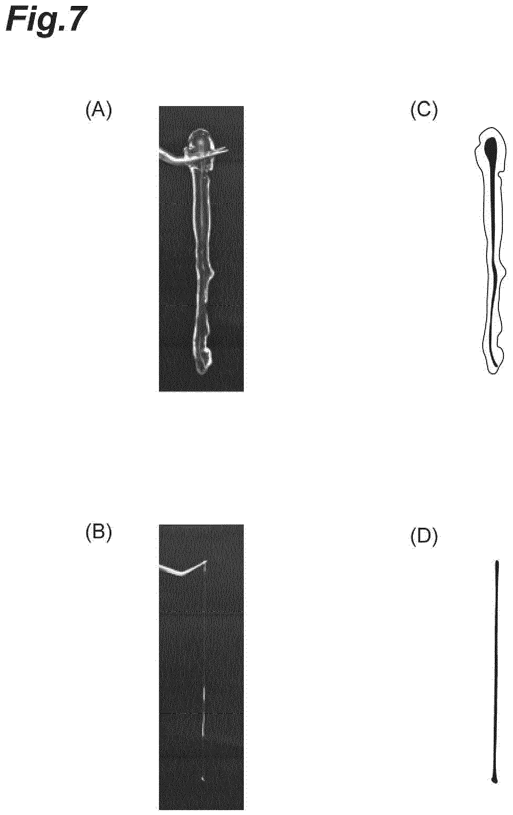

FIG. 7 is a photograph showing a polymer aggregate obtained in Example 1 (in which spider silk proteins aggregate to be formed in a fiber form) and a trace drawing which traces the photograph. FIG. 7(A) is a photograph showing a polymer aggregate formed in a gel. FIG. 7(B) is a photograph showing the polymer aggregate from which the gel is removed. FIG. 7(C) is a trace drawing which traces the photograph of FIG. 7(A). FIG. 7(D) is a trace drawing which traces the photograph of FIG. 7(B).

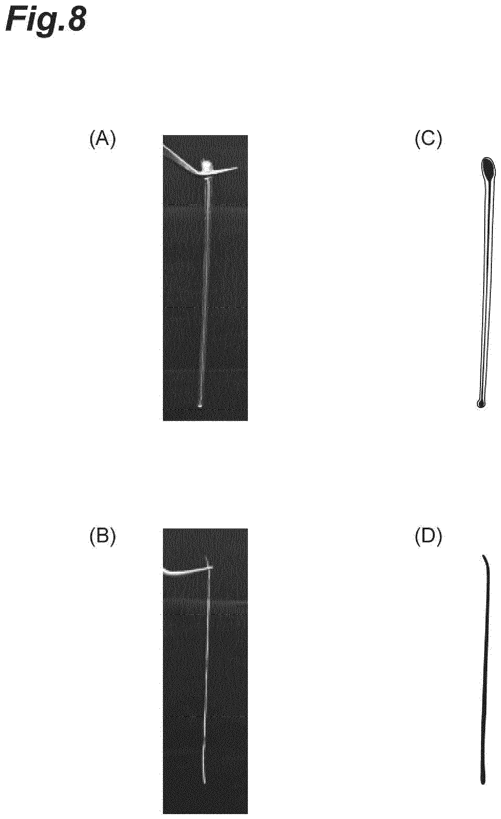

FIG. 8 is a photograph showing a polymer aggregate obtained in Example 2 (in which spider silk proteins aggregate to be formed in a fiber form) and a trace drawing which traces the photograph. FIG. 8(A) is a photograph showing a polymer aggregate formed in a gel. FIG. 8(B) is a photograph showing the polymer aggregate from which the gel is removed. FIG. 8(C) is a trace drawing which traces the photograph of FIG. 8(A). FIG. 8(D) is a trace drawing which traces the photograph of FIG. 8(B).

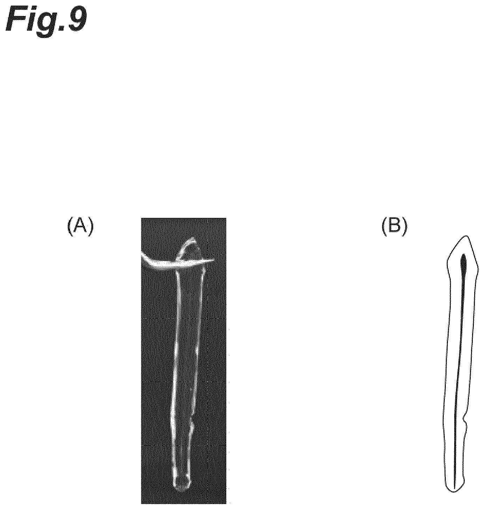

FIG. 9 is a photograph showing a polymer aggregate obtained in Example 3 (in which silk proteins aggregate to be formed in a fiber form) and a trace drawing which traces the photograph. FIG. 9(A) is a photograph showing a polymer aggregate formed in a gel. FIG. 9(B) is a trace drawing which traces the photograph of FIG. 9(A).

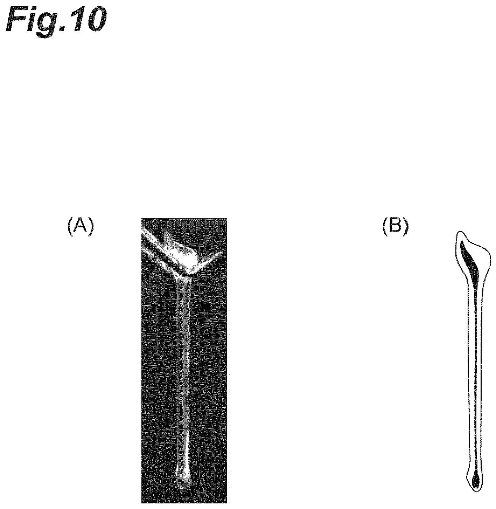

FIG. 10 is a photograph showing a polymer aggregate obtained in Example 4 (in which silk proteins aggregate to be formed in a fiber form) and a trace drawing which traces the photograph. FIG. 10(A) is a photograph showing a polymer aggregate formed in a gel. FIG 10(B) is a trace drawing which traces the photograph of FIG. 10(A).

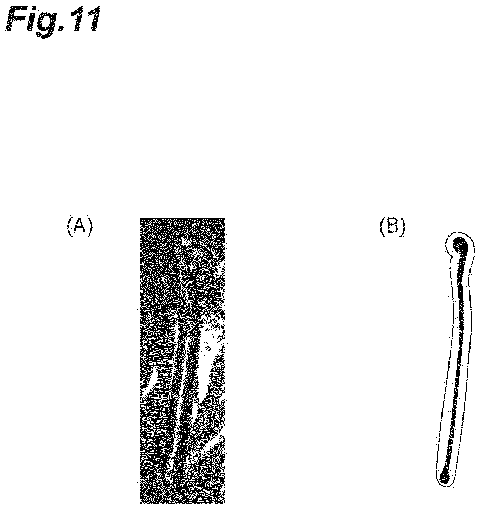

FIG. 11 is a photograph showing a polymer aggregate obtained in Example 6 (in which gelatin proteins aggregate to be formed in a fiber form) and a trace drawing which traces the photograph. FIG 11(A) is a photograph showing a polymer aggregate formed in a gel. FIG. 11(B) is a trace drawing which traces the photograph of FIG. 11(A).

### Embodiments for Carrying Out the Invention

Hereinafter, preferred embodiments of the present invention will be described in detail with reference to the drawings in some cases; however, the present invention is not limited to the following embodiments. Incidentally, in the drawings, the same or equivalent parts are attached with the same signs, and duplicate descriptions are appropriately omitted.

A method for producing a polymer aggregate according to the present invention includes a step for bringing a polymer solution containing a polymer substance and cations for gel formation into contact with an anion solution containing anions for gel formation to form a gel at an interface between the polymer solution and the anion solution (hereinafter, also referred to as "gel formation step"), and a step for causing the polymer substance to aggregate inside the gel (hereinafter, also referred to as "aggregation step").

### (Polymer Solution)

The polymer substance serving as a raw material is not particularly limited, and an arbitrary polymer substance can be used. Specific examples of the polymer substance include biological polymers such as proteins, nucleic acids, lipids, and polysaccharides (for example, cellulose, starch, and the like) and synthetic polymers such as synthetic resins (for example, polyvinyl chloride, polyethylene, phenolic resin, and the like), silicon resins (for example, silicon rubber, and the like), synthetic fibers (for example, nylon, vinylon, polyester, polyethylene terephthalate, and the like), and synthetic rubbers (for example, butadiene rubber, isoprene rubber, and the like).

Since biological polymers such as proteins have a room for improvement particularly in the production process of the polymer aggregate, the production method according to the present invention is suitably applied to the biological polymers such as proteins. The type of protein is not limited, and an arbitrary protein can be applied. The protein serving as a raw material may be produced using microorganisms or the like by genetic engineering technology, may be produced by synthesis, or may be produced by purifying a naturally derived protein.

The protein is preferably a structural protein. The structural protein is a protein having a role of constructing a living structure and is different from a functional protein such as enzyme, hormone, or antibody. Examples of the structural protein may include natural structural proteins such as fibroin, collagen, resilin, elastin, and keratin that occur in nature. As naturally occurring fibroin, fibroins produced by insects and spiders are known. In this embodiment, the structural protein preferably includes spider silk proteins. Incidentally, among structural proteins, at least one selected from a polypeptide (spider silk fibroin) derived from a spider silk protein, a silk protein (silken thread, silk fibroin, or the like), gelatin, collagen, and elastin is preferable. These proteins are easily formed in various forms including a fiber.

Examples of fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and hornet silk proteins discharged from larvae of Vespa simillima xanthoptera.

As a more specific example of fibroin produced by insects, for example, Bombyx mori fibroin L chain (GenBank Accession No.: M76430 (base sequence), AAA27840.1 (amino acid sequence)) is exemplified.

The silken thread is a fiber obtained from a cocoon produced by a silkworm, which is a larva of Bombyx mori (cocoon filament). Two fibroins are covered with a sticky substance (sericin) to form one cocoon filament. The fibroin is composed of a plurality of fibrils, and the outer side of the fibroin is covered with the sericin having four layers, thereby configuring one cocoon filament.

The silk fibroin is obtained by using a natural or domesticated cocoon or a used or waste silk cloth as a raw material, removing sericin covering the fibroin and other substances such as fat therefrom, and purifying the fibroin. The silk fibroin is preferably silk fibroin obtained by forming the purified fibroin into freeze-dried powder.

The spider silk fibroin is fibroin produced by spiders. A spider has a maximum of seven types of silk gland, and the silk glands produce fibroins (spider silk proteins) each having a different property, respectively. The spider silk proteins are designated, according to organs of origins thereof, as a major ampullate spider protein (MaSp) having high toughness, a minor ampullate spider protein (MiSp) having a high level of extension force, and flagelliform (Flag), tubuliform, aggregate, aciniform, and pyriform spider silk proteins.

Examples of the fibroin produced by spiders include spider silk proteins produced by spiders belonging to the genus Araneus such as Araneus ventricosus, Araneus diadematus, Araneus quadratus, Araneus pentagrammicus, and Araneus nojimai, spiders belonging to the genus Neoscona such as Neoscona scylla, Neoscona nautica, Neoscona adianta, and Neoscona scylloides, spiders belonging to the genus Pronus such as Pronous minutus, spiders belonging to the genus Cyrtarachne such as Cyrtarachne bufo and Cyrtarachne inaequalis, spiders belonging to the genus Gasteracantha such as Gasteracantha kuhlii and Gasteracantha mammosa, spiders belonging to the genus Ordgarius such as Ordgarius hobsoni and Ordgarius sexspinosus, spiders belonging to the genus Argiope such as Argiope amoena, Argiope minuta, and Argiope bruennichi, spiders belonging to the genus Arachnura such as Arachnura logio, spiders belonging to the genus Acusilas such as Acusilas coccineus, spiders belonging to the genus Cytophora such as Cyrtophora moluccensis, Cyptoployd extMerragtica, and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata, and Cyclosa atrata, and spiders belonging to the genus Chorizopes such as Chorizopes nipponicus; and spider silk proteins produced by spiders belonging to the Tetragnathidae family including spiders belonging to the genus Tetragnatha such as Tetragnatha praedonia, Tetragnatha maxillosa, Tetragnatha extensa, and Tetragnatha squamata, spiders belonging to the genus Leucauge such as Leucauge magnifica, Leucauge blanda, and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Pachygnatha tenera, spiders belonging to the genus Latrodectus such as Latrodectus mactans, Latrodectus hasseltii, Latrodectus geometricus, and Latrodectus tredecimguttatus, spiders belonging to the genus Euprosthenops, and the like. Examples of the spider silk proteins include MaSp (MaSp1 and MaSp2), dragline proteins such as ADF (ADF3 and ADF4), and MiSp (MiSp1 and MiSp2).

More specific examples of the fibroin produced by spiders include fibroin-3 (adf-3) [derived from Araneus diadematus] (GenBank Accession No.: AAC47010 (amino acid sequence), U47855 (base sequence)), fibroin-4 (adf-4) [derived from Araneus diadematus] (GenBank Accession No.: AAC47011 (amino acid sequence), U47856 (base sequence)), dragline silk protein spidroin 1 [derived from Nephila clavipes] (GenBank Accession No.: AAC04504 (amino acid sequence), U37520 (base sequence)), major ampullate spidroin 1 [derived from Latrodectus hesperus] (GenBank Accession No.: ABR68856 (amino acid sequence), EF595246 (base sequence)), dragline silk protein spidroin 2 [derived from Nephila clavata] (GenBank Accession No.: AAL32472 (amino acid sequence), AF441245 (base sequence)), major ampullate spidroin 1 [derived from Euprosthenops australis] (GenBank Accession No.: CAJ00428 (amino acid sequence), AJ973155 (base sequence)), and major ampullate spidroin 2 [Euprosthenops australis] (GenBank Accession No.: CAM32249.1 (amino acid sequence), AM490169 (base sequence)), minor ampullate silk protein 1 [Nephila clavipes] (GenBank Accession No.: AAC14589.1 (amino acid sequence)), minor ampullate silk protein 2 [Nephila clavipes] (GenBank Accession No.: AAC14591.1 (amino acid sequence)), and minor ampullate spidroin-like protein [Nephilengys cruentata] (GenBank Accession No.: ABR37278.1 (amino acid sequence).

More specific examples of the naturally derived fibroin may further include fibroins whose sequence information is registered in NCBI GenBank. For example, this can be confirmed by extracting, from sequences including INV as DIVISION of the sequence information registered in NCBI GenBank, sequences described as a keyword of spidroin, ampullate, fibroin, "silk and polypeptide," or "silk and protein" in DEFINITION, and sequences described by character strings of specific products from CDS and specific character strings in TISSUE TYPE from SOURCE.

The protein may be a polypeptide derived from the natural protein, that is, a recombinant polypeptide. For example, the recombinant fibroin is produced in several foreign protein production systems, and as the production method therefor, transgenic goats, transgenic silkworms, or recombinant plants or mammalian cells are used.

The recombinant fibroin can also be obtained by designing an amino acid sequence of naturally derived fibroin or an amino acid sequence, which corresponds to modification of an amino acid sequence corresponding to substitution, deletion, insertion and/or addition of one or plural amino acid residues to an amino acid sequence of naturally derived fibroin, and chemically synthesizing a nucleic acid encoding the designed amino acid sequence.

The recombinant polypeptide of major dragline silk protein can be represented, for example, as a protein containing a domain sequence represented by Formula 1: [(A)ₙ motif-REP]ₘ (here, in Formula 1, (A)ₙ motif represents an amino acid sequence composed of 4 to 20 amino acid residues, and the number of alanine residues to the number of whole amino acid residues in the (A)ₙ motif is 70% or more; REP represents an amino acid sequence composed of 10 to 200 amino acid residues; m represents an integer of 5 to 300; a plurality of (A)ₙ motifs may be the same or different amino acid sequence from each other; and a plurality of REPs may be the same or different amino acid sequence from each other.). Specifically, proteins containing amino acid sequences represented by SEQ ID NOs: 1 to 3 and SEQ ID NOs: 8 and 9 can be exemplified.

Examples of the recombinant polypeptide of collagen may include proteins containing a domain sequence represented by Formula 2: [REP2]ₒ (here, in Formula 2, o represents an integer of 5 to 300; REP2 represents an amino acid sequence composed of Gly-X-Y; X and Y represent an arbitrary amino acid residue other than Gly; and a plurality of REP2s may be the same or different amino acid sequence from each other.). Specifically, a protein containing an amino acid sequence represented by SEQ ID NO: 10 can be exemplified. The amino acid sequence represented by SEQ ID NO: 10 is an amino acid sequence obtained by adding an amino acid sequence represented by SEQ ID NO: 4 to the N-terminal of a partial sequence of human collagen type 4 obtained from the NCBI database (NCBI Genbank Accession No.: CAA56335.1, GI: 3702452), specifically, an amino acid sequence thereof from the 301st residue to the 540th residue that corresponds to repetitive sections and motifs in the partial sequence of human collagen type 4.

Examples of the recombinant polypeptide of resilin may include proteins containing a domain sequence represented by Formula 3: [REP3]ₚ (here, in Formula 3, p represents an integer of 4 to 300; REP3 represents an amino acid sequence composed of Ser-J-J-Tyr-Gly-U-Pro; J represents an arbitrary amino acid residue, and particularly, is preferably an amino acid residue selected from the group consisting of Asp, Ser, and Thr; U represents an arbitrary amino acid residue, and particularly, is preferably an amino acid residue selected from the group consisting of Pro, Ala, Thr, and Ser; and a plurality of REP3s may be the same or different amino acid sequence from each other.). Specifically, a protein containing an amino acid sequence represented by SEQ ID NO: 11 can be exemplified. The amino acid sequence represented by SEQ ID NO: 11 is an amino acid sequence obtained as follows: in an amino acid sequence of resilin (NCBI Genbank Accession No.: NP 611157, Gl: 24654243), the 87th residue Thr and the 95th residue Asn are substituted with Ser and Asp, respectively, and an amino acid sequence represented by SEQ ID NO: 4 is added to the

N-terminal of an amino acid sequence from the 19th residue to the 321st residue of the substituted sequence.

Examples of the recombinant polypeptide of elastin may include proteins containing amino acid sequences of NCBI Genbank Accession Nos.: AAC98395 (human), 147076 (sheep), NP786966 (bovine), and the like. Specifically, a protein containing an amino acid sequence represented by SEQ ID NO: 12 can be exemplified. The amino acid sequence represented by SEQ ID NO: 12 is amino acid sequence obtained by adding an amino acid sequence represented by SEQ ID NO: 4 to the N-terminal of an amino acid sequence from the 121st residue to the 390th residue of the amino acid sequence of NCBI Genbank Accession No.: AAC98395.

Examples of the recombinant polypeptide of keratin may include Capra hircus type I keratin. Specifically, a protein containing an amino acid sequence represented by SEQ ID NO: 13 (amino acid sequence of NCBI Genbank Accession No.: ACY30466) can be exemplified.

The recombinant polypeptide may be a recombinant fibroin containing an amino acid sequence having a sequence identity of 90% or more with the amino acid sequence of the above-described fibroin.

The aforementioned recombinant fibroin may contain a tag sequence at either or both of the N-terminal and the C-terminal. According to this, isolation, immobilization, detection, visualization, and the like of the recombinant fibroin are enabled.

As the tag sequence, for example, an affinity tag using specific affinity (affinity) with other molecules can be exemplified. As a specific example of the affinity tag, a histidine tag (His tag) can be exemplified. The His tag is a short peptide containing 4 to 10 histidine residues arranged and has a property that specifically binds to metal ions such as nickel. Thus, the His tag can be used for isolation of recombinant fibroin by chelating metal chromatography. As a specific example of the tag sequence, for example, an amino acid sequence represented by SEQ ID NO: 4 is exemplified.

Further, a tag sequence such as glutathione-S-transferase (GST) specifically binding to glutathione or maltose-binding protein (MBP) specifically binding to maltose can also be used.

Furthermore, an "epitope tag" using antigen-antibody reaction can also be used. By adding peptide (epitope) exhibiting antigenicity as a tag sequence, an antigen can be bound to the epitope. Examples of the epitope tag may include a HA (peptide sequence of hemagglutinin of influenza virus) tag, a myc tag, and a FLAG tag. By using the epitope tag, recombinant fibroin can be easily purified with high specificity.

Further, one obtained by isolating a tag sequence with a specific protease can also be used. By performing protease treatment to a protein adsorbed through the tag sequence, recombinant fibroin from which the tag sequence is isolated can also be collected.

A method for producing a protein by the genetic recombination technology will be described below. Such a protein can be produced, for example, by expressing a nucleic acid by a host transformed by an expression vector which has a nucleic acid sequence encoding the protein and one or a plurality of regulatory sequences operably linked to the nucleic acid sequence.

A method for producing a gene encoding a protein is not particularly limited. For example, a gene can be produced by a method of using a gene encoding a natural structural protein and amplifying and cloning the gene by polymerase chain reaction (PCR) or the like or can be produced by chemical synthesis. The method of chemically synthesizing a gene is also not particularly limited, and for example, a gene can be chemically synthesized by a method of linking oligonucleotide, which is automatically synthesized by AKTA oligopilot plus 10/100 (GE healthcare Japan, Ltd.) or the like, by PCR or the like based on amino acid sequence information of structural protein obtained from NCBI web database or the like. At this time, in order to facilitate purification and confirmation of a protein, a gene encoding a protein, which is composed of an amino acid sequence obtained by adding an amino acid sequence composed of the start codon and the His 10-tag to the N-terminal of the amino acid sequence, may be synthesized.

The regulatory sequence is a sequence regulating expression of a recombinant protein in a host (for example, a promoter, an enhancer, a ribosome binding sequence, a transcription termination sequence, or the like), and can be appropriately selected according to the type of host. As a promoter, an inducible promoter which functions in host cells and is capable of inducing expression of a target protein, may be used. The inducible promoter is a promoter which can control transcription in the presence of an inducing substance (expression inducer), in the absence of a repressor molecule, or by a physical factor such as an increase or decrease in temperature, osmotic pressure or pH value.

As for the type of the expression vector, a plasmid vector, a virus vector, a cosmid vector, a fosmid vector, an artificial chromosome vector, and the like can be appropriately selected depending on the type of the host. As the expression vector, those capable of autonomous replication or integration into the chromosome in the host and containing a promoter at a position where a nucleic acid encoding a target protein can be transcripted are preferably used.

As the host, any of prokaryotes and eukaryotes such as yeasts, filamentous fungi, insect cells, animal cells, and plant cells can be preferably used.

Preferable examples of prokaryotes may include bacteria belonging to the genera Escherichia, Brevibacillus, Serratia, Bacillus, Microbacterium, Brevibacterium, Corynebacterium, Pseudomonas, and the like.

Examples of a vector, which introduces a nucleic acid encoding a protein, may include pBTrp2 (manufactured by Boehringer Mannheim GmbH), pGEX (manufactured by Pharmacia), and pUC18, pBluescriptII, pSupex, pET22b, pCold, pUB110, and pNCO2 (Japanese Unexamined Patent Publication No. 2002-238569).

Examples of hosts of eukaryotes may include yeasts and filamentous fungi (fungi and the like).

Examples of the yeasts may include yeasts belonging to the genera Saccharomyces, Pichia, Schizosaccharomyces, and the like. Examples of the filamentous fungi may include filamentous fungi belonging to the genera Aspergillus, Penicillium, Trichoderma, and the like.

Examples of the vector may include YEP13 (ATCC37115) and YEp24 (ATCC37051).

As a method of introducing the expression vector into the host cell, any method can be used as long as it is a method of introducing DNA into the host cell. Examples of the introduction method may include a method using calcium ions [Proc. Natl. Acad. Sci. USA, 69, 2110 (1972)], an electroporation method, a spheroplast method, a protoplast method, a lithium acetate method, and a competent method.

As the method of expressing a nucleic acid by a host transformed by an expression vector, other than the direct expression, production by secretion, expression of fusion protein, or the like can be performed according to the method described in Molecular Cloning, 2nd edition, or the like.

A protein can be produced, for example, by culturing a host transformed by an expression vector in an incubation medium, growing and accumulating the protein in the incubation medium, and collecting the protein from the incubation medium. The method of culturing the host in the incubation medium can be performed according to a method generally used in culturing of a host.

In a case where a host is prokaryotes such as Escherichia coli or eukaryotes such as yeast, as an incubation medium of the host, either natural or synthetic culture medium may be used as long as it contains a carbon source, a nitrogen source, inorganic salts, and the like that may be assimilated by the host, and culturing of the host is efficiently carried out.

The carbon source may be any that may be assimilated by the host, and for example, glucose, fructose, sucrose, molasses containing these, carbohydrates such as starch and starch hydrolysate, organic acids such as acetic acid and propionic acid, and alcohols such as ethanol and propanol can be used.

As the nitrogen source, for example, ammonia, an ammonium salt of inorganic acids or organic acids such as ammonium chloride, ammonium sulfate, ammonium acetate, and ammonium phosphate, other nitrogen-containing compounds, as well as peptone, meat extract, yeast extract, corn steep liquor, casein hydrolysate, soybean cake and soybean cake hydrolysate, and various zymocytes and digests thereof can be used.

As inorganic salts, for example, monopotassium phosphate, dipotassium phosphate, magnesium phosphate, magnesium sulfate, sodium chloride, ferrous sulfate, manganese sulfate, copper sulfate, and calcium carbonate can be used.

The culturing of prokaryotes such as Escherichia coli or eukaryote such as yeast can be carried out, for example, under aerobic conditions such as shaking culture or deep aeration stirring culture. The culture temperature is, for example, 15 to 40°C. The culture time is typically 16 hours to 7 days. The pH of the incubation medium during cultivating is preferably maintained to 3.0 to 9.0. The adjustment of pH of the incubation medium can be performed using an inorganic acid, an organic acid, an alkali solution, urea, calcium carbonate, ammonia, or the like.

Further, antibiotic such as ampicillin and tetracycline may also be added to an incubation medium as necessary during cultivating. When cultivating a microorganism transformed with an expression vector using an inducible promoter as the promoter, an inducer may be added to the culture medium as necessary. For example, when cultivating a microorganism transformed with an expression vector using a lac promoter, isopropyl-β-D-thingalactopyranoside or the like may be added, and when cultivating a microorganism transformed with an expression vector using a trp promoter, indoleacrylic acid or the like may be used.

Isolation and purification of the protein can be performed by a method generally used. For example, in a case where a protein is expressed in a dissolved state within the cell, after the culturing is complete, host cells are recovered by centrifugation, suspended in an aqueous buffer solution, and then the host cells are disrupted with a sonicator, a french press, Manton-Gaulin homogenizer, Dynomill, or the like to obtain a cell-free extract. From the supernatant obtained by centrifuging the cell-free extract, a method generally used for isolation and purification of the protein, that is, methods such as a solvent extraction method, a salt precipitation method by ammonium sulfate or the like, a desalting method, a precipitation method by an organic solvent, diethylaminoethyl (DEAE)-Sepharose, an anion exchange chromatography method using a resin such as DIAION HPA-75 (manufactured by Mitsubishi Chemical Corporation), a cation exchange chromatography method using a resin such as S-Sepharose FF (manufactured by Pharmacia), a hydrophobic chromatography method using a resin such as butyl Sepharose or phenyl Sepharose, a gel filtration method using a molecular sieve, an affinity chromatography method, a chromatofocusing method, and an electrophoresis method such as isoelectric electrophoresis are used alone or in combination, and a purified preparation can be obtained.

Further, in a case where the protein is expressed by forming an insoluble form within cells, similarly, the host cells are disrupted after recovery and centrifuged to recover the insoluble form of the modified fibroin as a precipitated fraction. The insoluble form of the modified fibroin thus recovered can be dissolved with a protein denaturant. After the operation, a purified preparation of the modified fibroin can be obtained by the isolation and purification method similar to the above.

In a case where a protein is secreted outside the cell, the protein can be recovered from the culture supernatant. That is, the culture is treated by a method such as centrifugation to obtain the culture supernatant, and a purified preparation can be obtained from the culture supernatant by an isolation and purification method similar to the above.

The cations for gel formation are cations which bind to the anions for gel formation to form a gel. The cations for gel formation are typically multivalent cations and hydrogen ions (protons). The multivalent cation is a compound having a positive charge of divalent or more, and for example, a divalent or more metal cation and a compound having two or more cationic groups in one molecule are exemplified. As the compound having two or more cationic groups in one molecule, for example, an ion of polyamine having two or more amino groups in one molecule is exemplified. Examples of the divalent or more metal cation include alkaline-earth metal ions such as Ca²⁺ and Mg²⁺; transition metal ions such as Mn²⁺, Fe²⁺, Fe³⁺, Co²⁺, and Cu²⁺; Zn²⁺; and Al³⁺.

The anions for gel formation are anions which bind to the cations for gel formation to form a gel. Examples of the anions for gel formation include ions of polysaccharides having an anionic group such as carrageenan, pectin (for example, LM pectin or the like), gum arabic, xanthane gum, gellan gum, soybean polysaccharides, and alginic acid (for example, sodium alginate) and ions of acids such as polylactic acid and polyphosphoric acid.

The cations for gel formation and the anions for gel formation can be used arbitrarily in combination, but from the viewpoint of having excellent gel formation efficiency, it is preferable that the cations for gel formation are divalent or higher metal cations and the anions for gel formation are alginate ions, and it is more preferable that the cations for gel formation are Ca²⁺ and the anions for gel formation are alginate ions.

The polymer solution (dope) can be obtained, for example, by adding a polymer substance to a solvent having cations for gel formation dissolved therein, and dissolving the polymer substance by a known method such as stirring, heating, and electric field application.

The cations for gel formation may be added as a compound generating the cations for gel formation when being dissolved in a solvent (multivalent cation compound) to the solvent. Specific examples of the multivalent cation compound include metallic halide (for example, CaCl₂, MgCl₂), metallic nitrate (for example, Ca(NO₃)₂, Mg(NO₃)₂), metal thiocyanate (for example, Ca(SCN)₂, Mg(SCN)₂), a diamine compound (for example, ethylenediamine, p-phenylenediamine), and a multivalent complex salt (for example, [Co₄(OH)₆·en₆](NO₃)₆).

The solvent of the polymer solution may be an aqueous solvent such as water and a buffer solution, and may be an organic solvent such as dimethylsulfoxide (DMSO), N,N-dimethylformamide (DMF), hexafluoroisopropanol (HFIP), and hexafluroacetone (HFAc). The solvent of the polymer solution may be a mixed solvent obtained by mixing two or more kinds of solvent. The mixed solvent also includes a mixed solvent of an aqueous solvent and an organic solvent.

The polymer solution may further contain a dissolution promoter which promotes dissolution of the polymer substance. According to this, the polymer solution is easily prepared. As a specific example of the dissolution promoter, for example, in a case where the polymer substance is a protein, for example, inorganic salts composed of Lewis acids and Lewis bases described below are exemplified. Examples of the Lewis bases include oxo-acid ions (such as nitrate ion and perchlorate ion), metal oxo-acid ions (such as permanganate ion), halide ions, thiocyanate ions, and cyanate ions. Examples of the Lewis acids include metal ions such as alkali metal ions and alkaline-earth metal ions, polyatomic ions such as ammonium ions, and complex ions. Specific examples of the inorganic salts composed of Lewis acids and Lewis bases include lithium salts such as lithium chloride, lithium bromide, lithium iodide, lithium nitrate, lithium perchlorate, and lithium thiocyanate, calcium salts such as calcium chloride, calcium bromide, calcium iodide, calcium nitrate, calcium perchlorate, and calcium thiocyanate, iron salts such as iron chloride, iron bromide, iron iodide, iron nitrate, iron perchlorate, and iron thiocyanate, aluminum salts such as aluminum chloride, aluminum bromide, aluminum iodide, aluminum nitrate, aluminum perchlorate, and aluminum thiocyanate, potassium salts such as potassium chloride, potassium bromide, potassium iodide, potassium nitrate, potassium perchlorate, and potassium thiocyanate, sodium salts such as sodium chloride, sodium bromide, sodium iodide, sodium nitrate, sodium perchlorate, and sodium thiocyanate, zinc salts such as zinc chloride, zinc bromide, zinc iodide, zinc nitrate, zinc perchlorate, and zinc thiocyanate, magnesium salts such as magnesium chloride, magnesium bromide, magnesium iodide, magnesium nitrate, magnesium perchlorate, and magnesium thiocyanate, barium salts such as barium chloride, barium bromide, barium iodide, barium nitrate, barium perchlorate, and barium thiocyanate, and strontium salts such as strontium chloride, strontium bromide, strontium iodide, strontium nitrate, strontium perchlorate, and strontium thiocyanate. In a case where the polymer substance is vulcanized rubber, specific examples of the dissolution promoter include thiol and amine. In a case where the polymer substance is cellulose, specific examples of the dissolution promoter include carbon disulfide and sodium hydroxide. In a case where the polymer substance is a synthetic resin, for example, an acrylic resin, specific examples of the dissolution promoter include sodium thiocyanate, in a case where the polymer substance is polyamide and polyparaphenylene terephthalamide, specific examples of the dissolution promoter include sulfuric acid, and in a case where the polymer substance is polyvinyl chloride, specific examples of the dissolution promoter include acetone and cyclohexane. Incidentally, depending on a combination of the polymer substance and the cations for gel formation, the cations for gel formation may act as a dissolution promoter of promoting dissolution of the polymer substance.

The content of the cations for gel formation in the polymer solution is appropriately set according to types and the like of the polymer substance, the cations for gel formation, the anions for gel formation, the solvent, and other additives. For example, in a case where the polymer substance is a protein, the cations for gel formation are Ca²⁺, and the anions for gel formation are alginate ions, the content of the cations for gel formation is preferably in a concentration range of 0.1 to 5.0 mol/L and more preferably in a concentration range of 0.4 to 3.0 mol/L.

The content of the polymer substance in the polymer solution is appropriately set according to the type of the polymer substance, and the like. For example, in a case where the polymer substance is a protein, the content of the polymer substance is preferably 10 to 50 mass% and more preferably 15 to 30 mass% based on the total amount of the polymer solution.

The viscosity of the polymer solution is not particularly limited, but for example, from the viewpoint of facilitating molding, the viscosity is preferably 50 to 20000 cP and more preferably 200 to 8000 cP at 25°C. The viscosity of the polymer solution is measured, for example, by an EMS viscometer.

### (Anion Solution)

The anion solution (coagulation liquid) can be obtained, for example, by adding the anions for gel formation to a solvent, and dissolving the anions for gel formation by a known method such as stirring, heating, and electric field application.

The anions for gel formation may be added as a compound generating the anions for gel formation when being dissolved in a solvent (anion compound) to the solvent. Specific examples of the anion compound include polysaccharides having an anionic group and salts thereof. Preferred specific examples of the polysaccharides having an anionic group and salts thereof include sodium alginate. The sodium alginate is represented by (NaC₆H₇O₆)ₙ (n is 1 to 5000), and is abbreviated as Na-ALG in some cases.

The solvent of the anion solution may be an aqueous solvent such as water and a buffer solution, and may be an organic solvent such as dimethylsulfoxide (DMSO), N,N-dimethylformamide (DMF), hexafluoroisopropanol (HFIP), and hexafluroacetone (HFAc). The solvent of the anion solution may be a mixed solvent obtained by mixing two or more kinds of solvent. The mixed solvent also includes a mixed solvent of an aqueous solvent and an organic solvent.

The solvent of the anion solution may be the same as the solvent of the polymer solution. In addition, both the solvents may be an aqueous solvent. When both the polymer solution (dope) and the anion solution (coagulation liquid) are set to an aqueous solvent, the polymer aggregate can be formed in a water system. According to this, the polymer aggregate thus obtained is applicable to a living body, and for example, can be used instead of silken threads currently used as surgical sewing threads. Moreover, the polymer aggregate is applicable to artificial blood vessels, artificial skins, artificial bones, and the like.

The anion solution may further contain an aggregation promoter for the polymer substance. According to this, the production efficiency of the polymer aggregate can be improved. As a specific example of the aggregation promoter for the polymer substance, for example, in a case where the polymer substance is a protein, alkali metal ions such as Na⁺, Li⁺, and K⁺ are exemplified. In a case where the polymer substance is cellulose, as a specific example of the aggregation promoter for the polymer substance, ethanol is exemplified. In a case where the polymer substance is non-vulcanized rubber, as a specific example of the aggregation promoter for the polymer, a disulfide compound is exemplified.

The content of the anions for gel formation in the anion solution is appropriately set according to types and the like of the polymer substance, the cations for gel formation, the anions for gel formation, the solvent, and other additives. For example, in a case where the polymer substance is a protein, the cations for gel formation are Ca²⁺, and the anions for gel formation are alginate ions, the content of the anions for gel formation is preferably in a concentration range of 0.1 to 6.0 weight% and more preferably in a concentration range of 1.0 to 2.0 weight%.

### (Gel Formation Step)

In the gel formation step, a polymer solution is brought into contact with an anion solution to form a gel at an interface between the polymer solution and the anion solution. When the polymer solution is brought into contact with the anion solution, by controlling the form of a contact surface, a gel in an arbitrary form such as a fibrous form, a film form, a hollow pipe form, and a bead form can be formed.

In the case of a fibrous form, for example, a gel can be molded by the polymer solution being continuously extruded from a spinning nozzle to the anion solution. In the case of a film form, for example, a gel can be molded by the polymer solution being continuously extruded from a slit hole to the anion solution. In the case of a hollow pipe form, for example, a gel can be molded by the polymer solution being continuously extruded from a hollow slit hole or through a double tube to the anion solution and the polymer solution also being injected to the inside of the polymer solution. In the case of a bead form, for example, a gel can be molded by the polymer solution being intermittently extruded from a nozzle to the anion solution.

### (Aggregation Step)

In the aggregation step, the polymer substance is aggregated inside the gel. In the aggregation step, the gel functions as a dialysis membrane, and for example, excessive cations for gel formation or dissolution promoter contained in the polymer solution is discharged through the gel into the anion solution so that aggregation of the polymer substance proceeds. Further, for example, the aggregation promoter contained in the anion solution infiltrates through the gel into the polymer solution so that aggregation of the polymer substance proceeds. In the aggregation step, for example, the anion solution may be stirred. According to this, the efficiency of substance transfer through the gel is improved, and thus production efficiency is improved.

The time for executing the aggregation step may be appropriately set according to types and the like of the polymer substance, the cations for gel formation, the anions for gel formation, the solvent, and other additives and the form, application, and the like of the polymer aggregate, but is typically 3 minutes to 3 hours and preferably 3 minutes to 2 hours.

By executing the aggregation step, a polymer aggregate to which the gel is bound in a sheath form is obtainable.

### (Aggregation Step 2)

The production method according to this embodiment may further include a step for immersing the polymer aggregate to which the gel is bound in a sheath form in a solvent such as water (also referred to as "aggregation step 2") after the aggregation step. According to this, movement of the cations for gel formation or the dissolution promoter from the inside of the gel to the solvent is promoted so that the polymer aggregate can be further aggregated.

The time for executing the aggregation step 2 may be appropriately set according to types and the like of the polymer substance, the cations for gel formation, the anions for gel formation, the solvent, and other additives and the form, application, and the like of the polymer aggregate, but is typically 3 minutes to 3 hours, preferably 3 minutes to 2 hours, and more preferably 3 minutes to 1 hour.

### (Gel Removal Step)

The production method according to this embodiment may further include a step for removing the gel from the polymer aggregate (also referred to as "gel removal step") after the aggregation step or the aggregation step 2. As for the polymer aggregate, the polymer aggregate to which the gel is bound in a sheath form may be provided without change according to the application, and in a case where the gel is unnecessary, the polymer aggregate from which the gel is removed by the gel removal step may be provided. As the method of removing the gel, for example, a removal method using a mechanical means such as crushing the gel using a roller, a chemical method of immersing the gel in a solution that does not dissolve the polymer aggregate but dissolves only the gel, or a method of removing the gel by ultrasonic wave or heat is exemplified.

The production method according to this embodiment will be further described with reference to FIGS. 1 to 6.

FIG. 1 is an explanatory diagram illustrating a production method according to an embodiment. In the production method illustrated in FIG. 1, a polymer aggregate in a fibrous form is obtained. FIG. 1(A) is an explanatory diagram illustrating a step for extruding a polymer solution (dope) into an anion solution (coagulation liquid). A dope 1 is put in a syringe 9a, and the dope 1 is continuously extruded from a nozzle to a coagulation liquid 4. The dope 1 is obtained by a polymer substance (for example, a polypeptide derived from a spider silk protein) being dissolved in an aqueous solution containing cations for gel formation (for example, Ca²⁺). The coagulation liquid 4 is an aqueous solution containing anions for gel formation (for example, alginate ions). The coagulation liquid 4 has been put in a container 3 and slowly stirred. An extruded dope 2 forms a gel 5 at an interface in the coagulation liquid 4. The polymer substance (for example, a polypeptide derived from a spider silk protein) aggregates inside the gel 5 to form a polymer aggregate 8. Since the gel 5 in a fibrous form is formed by extrusion, the polymer aggregate 8 thus obtained also has a fibrous form.

FIG. 1(B) is an explanatory diagram illustrating a step for immersing a polymer aggregate covered with a gel in water and further causing the polymer aggregate to aggregate. Water 7 has been put in a container 6 and slowly stirred. The polymer aggregate 8 covered with the gel 5 is immersed in the water 7 with the gel 5. According to this, the polymer aggregate 8 further aggregates. This polymer aggregate is dried to thereby obtain threads with high strength.

FIG. 2 is an explanatory diagram illustrating a production method according to another embodiment. In the production method illustrated in FIG. 2, a polymer aggregate in a particulate form (bead form) is obtained. The dope 1 is put in the syringe 9a, and the dope 1 is intermittently extruded from the nozzle to the coagulation liquid 4. The extruded dope 2 forms the gel 5 at the interface in the coagulation liquid 4. The polymer substance aggregates inside the gel 5 to form the polymer aggregate 8. Since the gel 5 in a particulate form is formed by intermittent extrusion, the polymer aggregate 8 thus obtained also has a particulate form.

FIG. 3 is an explanatory diagram illustrating a production method according to another embodiment. In the production method illustrated in FIG. 3, a polymer aggregate in a film form is obtained. The dope 1 is continuously extruded from a slit hole 9b to the coagulation liquid 4. The extruded dope 2 forms the gel 5 in the coagulation liquid 4. The polymer substance aggregates inside the gel 5 to form the polymer aggregate 8. Since the gel 5 in a film form is formed by extrusion, the polymer aggregate 8 thus obtained also has a film form.

FIG. 4(A) is an explanatory diagram illustrating a production method according to another embodiment. FIG. 4(B) is an enlarged view of a part surrounded by A in FIG. 4(A). In the production method illustrated in FIG. 4(A), a polymer aggregate in a hollow pipe form or tubular form is obtained. The dope 1 is continuously extruded to the coagulation liquid 4 from the syringe 9a through a circular slit hole 9b, which is provided at a tip of the nozzle, to have a tubular form. The extruded dope 2 forms the gel 5 in a tubular form in the coagulation liquid 4. The polymer substance aggregates inside the gel 5 to form the polymer aggregate 8. Since the gel 5 in a tubular form is formed by extrusion, the polymer aggregate 8 thus obtained also has a small-diameter hollow pipe form or tubular form.

FIG. 5 is a schematic cross-sectional view illustrating a coagulation liquid-gel-dope interface in a production method according to an embodiment. The mechanism in which a polymer aggregate is formed by the production method according to the present invention will be described using a schematic cross-sectional view 10 illustrating the coagulation liquid-gel-dope interface illustrated in FIG. 5. In FIG. 5, a polymer solution containing Ca(SCN)₂ and fibroin is used as the dope, and an anion solution containing sodium alginate is used as the coagulation liquid. A gel layer 12 is formed from Ca²⁺ (cations for gel formation) contained in the dope and alginate ions (ALGⁿ⁻: anions for gel formation) contained in the coagulation liquid. That is, ion-exchange reaction occurs between Ca²⁺-fibroin of a dope layer 11 and sodium alginate (Na⁺-ALGⁿ⁻) of a coagulation liquid layer 13 to generate Ca²⁺-ALGⁿ⁻, thereby obtaining a gel. Thus, the gel layer 12 is formed. The gel layer 12 is considered to function as a dialysis membrane. As a result, low-molecular weight SCN⁻, H₂O, and Na⁺ move through the gel layer 12, whereas the fibroin that is a high molecule remains inside the gel. Further, it is considered that solubility of the fibroin in the inside of the gel is degraded by outflow of SCN⁻ through the gel layer 12, and thus aggregation proceeds.

FIG. 6 is a production process diagram according to an embodiment. A dope 22 is extruded from a nozzle 23 of an extruder 21 in a P direction, an extruded dope 24 is immersed in a coagulation liquid 26 of a coagulation bath 25, a gel 27 thus obtained is peeled off, an aggregated fibroin 28 is further aggregated by being immersed in pure water 30 of a water bath 29, and then the aggregated fibroin is caused to pass through a dryer 31 to be solidified, thereby obtaining a yarn roll 32. In this way, fibroin threads can be produced by a series of processes. The fibroin threads has practically sufficient strength even in a state of unstretched threads, but may be further stretched.

### Examples

Hereinafter, the present invention will be described in more detail based on Examples. However, the present invention is not limited to the following Examples.

### [Example 1]

### 1. Preparation of Polypeptide Derived from Spider Silk Protein

### <Gene Synthesis>

### (1) Gene Synthesis of ADF3Kai

A partial amino acid sequence of ADF3 (GI No.: 1263287), which is one of two principal dragline silk proteins of Araneus diadematus, was obtained from NCBI web database, and synthesis of a gene encoding an amino acid sequence (SEQ ID NO: 2), which is obtained by adding an amino acid sequence (SEQ ID NO: 4) composed of a start codon, His 10-tag and HRV3C Protease (Human rhinovirus 3C Protease) recognition site to the N-terminal of the partial amino acid sequence of ADF3, was outsourced to GenScript, Inc. As a result, a pUC57 vector to which a gene of ADF3Kai having a base sequence represented by SEQ ID NO: 5 had been introduced was obtained (having an Nde I site immediately upstream of 5' terminal of the gene and an Xba I site immediately downstream of 5' terminal thereof). Thereafter, the gene was subjected to a restriction enzyme treatment with Nde I and EcoR I, and recombined into a pET22b(+) expression vector.

### (2) Gene Synthesis of ADF3Kai-Large

The half of the gene sequence of ADF3Kai on the 5' side (hereinafter, referred to as a sequence A) was amplified by the PCR reaction using ADF3Kai as a template, and a T7 promoter primer (SEQ ID NO: 14) and a Rep Xba I primer (SEQ ID NO: 15). The obtained DNA fragment of the sequence A was recombined into a pUC118 vector that had been subjected to the restriction enzyme treatment with Nde I and Xba I in advance using a Mighty Cloning Kit (manufactured by Takara Bio Inc.). Similarly, the half of the gene sequence of ADF3Kai on the 3' side (hereinafter, referred to as a sequence B) was amplified by the PCR reaction using ADF3Kai as a template, and an Xba I Rep primer (SEQ ID NO: 16) and a T7 terminator primer (SEQ ID NO: 17). The obtained DNA fragment of the sequence B was recombined into a pUC118 vector that had been subjected to the restriction enzyme treatment with Xba I and EcoR I in advance using the Mighty Cloning Kit (manufactured by Takara Bio Inc.). The pUC118 vector to which the sequence A had been introduced and the pUC118 vector to which the sequence B had been introduced were subjected to the restriction enzyme treatment with Xba I and EcoR I, respectively, and target DNA fragments of the sequences A and B were purified by gel cut. The DNA fragments A and B and the pET22b(+) that had been subjected to the restriction enzyme treatment with Nde I and EcoR I in advance were subjected to a ligation reaction and transformed into Escherichia coli DH5α. After confirmation of the insertion of the target DNA fragments by a colony PCR using a T7 promoter primer and a T7 terminator primer, plasmid was extracted from a colony where a target band size (3.6 kbp) was obtained, and the entire base sequence was checked by a sequence reaction using a 3130xl Genetic Analyzer (Applied Biosystems). Consequently, the construction of a gene of ADF3Kai-Large represented by SEQ ID NO: 6 was confirmed. The amino acid sequence of ADF3Kai-Large is as represented by SEQ ID NO: 3.

### (3) Gene Synthesis of ADF3Kai-Large-NRSH1

With a pET22b(+) vector to which the gene of ADF3Kai-Large obtained above had been introduced used as a template, through site-directed mutagenesis using a PrimeStar Mutagenesis Basal Kit (manufactured by Takara Bio Inc.), a codon GGC corresponding to the 1155th amino acid residue, i.e., glycine (Gly), in the amino acid sequence of ADF3Kai-Large (SEQ ID NO: 3) was mutated into a stop codon TAA, and a gene of ADF3Kai-Large-NRSH1 represented by SEQ ID NO: 7 was constructed on the pET22b(+). The accuracy of the introduction of the mutation was checked by the sequence reaction using the 3130xl Genetic Analyzer (Applied Biosystems). Incidentally, the amino acid sequence of ADF3Kai-Large-NRSH1 (hereinafter, also simply referred to as "NRSH1") is as represented by SEQ ID NO: 1.

### <Expression of Protein>

The pET22b(+) expression vector containing the gene sequence of NRSH1 obtained above was transformed into Escherichia coli Rosetta (DE3). The obtained single colony was cultured for 15 hours in 2 mL of an LB culture medium containing ampicillin. Thereafter, 1.4 mL of the culture solution was added to 140 mL of an LB culture medium containing ampicillin, and cultured to an OD₆₀₀ of 3.5 under the conditions of 37°C and 200 rpm. Next, the culture solution with the OD₆₀₀ of 3.5 was added to 7 L of a 2×YT culture medium containing ampicillin together with 140 mL of 50% glucose, and further cultured to the OD₆₀₀ of 4.0. Thereafter, isopropyl-β-thiogalactopyranoside (IPTG) was added to the obtained culture solution with the OD₆₀₀ of 4.0 such that the final concentration would be 0.5 mM, thereby inducing the expression of protein. After a lapse of two hours from the addition of IPTG, the culture solution was centrifuged and bacterial cells were collected. Protein solutions prepared from the culture solution before the addition of IPTG and after the addition of IPTG were each electrophoresed in a polyacrylamide gel. Consequently, a band having a target size (about 101.1 kDa) was observed with the addition of IPTG, and the expression of the target protein was confirmed.

### <Preparation of Polypeptide>

(I) About 4.5 g of bacterial cells of Escherichia coli expressing the NRSH1 protein and 30 mL of a buffer solution AI (20 mM Tris-HCl, pH 7.4) were added to a centrifuge tube (50 mL). After dispersing the bacterial cells with a mixer ("SI-0286" manufactured by GE, level 10), the dispersion was centrifuged (10,000 rpm, 10 minutes, room temperature) with a centrifuge ("MX-305" manufactured by TOMY SEIKO CO., LTD.), and a supernatant was discarded.
(II) To a precipitate (bacterial cells) obtained by the centrifugation, 30 mL of the buffer solution AI and 0.3 mL of 0.1 M PMSF (dissolved by isopropanol) were added, and the precipitate was dispersed for 3 minutes with the mixer (level 10) manufactured by GE. Thereafter, the bacterial cells were disrupted using a sonicator ("VCX 500" manufactured by Sonics & Materials, Inc.) and centrifuged (10,000 rpm, 10 minutes, room temperature).
(III) To the precipitate obtained by the centrifugation, 30 mL of the buffer solution AI was added, and the precipitate was dispersed for 3 minutes with a mixer ("T 18 basic ULTRA-TURRAX" manufactured by IKA, level 2). Thereafter, the dispersion was centrifuged (10,000 rpm, 10 minutes, room temperature) with the centrifuge manufactured by TOMY SEIKO CO., LTD., and a supernatant was removed.
(IV) To the centrifuge tube from which a supernatant is discarded, 7.5 M of a urea buffer solution I (7.5 M urea, 10 mM sodium dihydrogen phosphate, 20 mM NaCl, 1 mM Tris-HCl, pH 7.0) was added, the precipitate was dispersed well with the sonicator ("VCX 500" manufactured by Sonics & Materials, Inc.) (level 7). Thereafter, the dispersion was dissolved for 120 minutes (200 rpm, 60°C) with a shaker ("BR-43FL/MR" manufactured by TAITEC CORPORATION). The protein solution after being dissolved was centrifuged (11,000×g, 10 minutes, room temperature) with the centrifuge manufactured by TOMY SEIKO CO., LTD., a supernatant was dialyzed with water using a dialysis tube (cellulose tube 36/32 manufactured by Sanko Junyaku Co., Ltd.). The white aggregated protein obtained after the dialysis was collected by centrifugation, and moisture was removed by a freeze dryer to collect freeze-dried powder. The purification degree of the target protein NRSH1 (about 101.1 kDa) in the obtained freeze-dried powder was checked by analyzing images of the results of polyacrylamide gel electrophoresis (CBB staining) of the protein powder using Totallab (nonlinear dynamics ltd.). As a result, the purification degree of NRSH1 was about 85%.

### 2. Preparation of Polymer Solution (Dope)

The spider silk protein (NRSH1) was added to a pure water solution of 1 M Ca(SCN)₂ to have a concentration of 20 mass%, and dissolved for 3 hours using a shaker. Thereafter, dusts and bubbles were removed to obtain a dope. The solution viscosity of the dope was 2088 cP (centipoises) at 25°C.

### 3. Preparation of Anion Solution (Coagulation Liquid)

Pure water containing 1.0 mass% of sodium alginate (Na-ALG) was used as a coagulation liquid.

### 4. Production of Polymer Aggregate

As illustrated in FIG. 1(A), a dope was extruded using a syringe to a coagulation liquid. The diameter of an extruding nozzle was set to 0.5 mm and the extruding speed was set to 50 mL/Hr. After extruding, the dope was immersed for 90 minutes in the coagulation liquid (gelling bath immersion time) to obtain a polymer aggregate formed in the gel. Thereafter, the gel covering the polymer aggregate was removed to obtain filamentous fibroin.

The production conditions and results are presented in Table 1 and FIG. 7. FIG. 7 is a photograph showing a polymer aggregate obtained in Example 1 (in which spider silk proteins aggregate to be formed in a fiber form) and a trace drawing which traces the photograph. FIG. 7(A) is a photograph showing a polymer aggregate formed in a gel. FIG. 7(B) is a photograph showing the polymer aggregate from which the gel is removed. FIG. 7(C) is a trace drawing which traces the photograph of FIG. 7(A). FIG. 7(D) is a trace drawing which traces the photograph of FIG. 7(B).

### [Example 2]

A polymer aggregate was produced in a similar manner to Example 1, except that a dope was prepared by adding the spider silk protein (NRSH1) to a DMSO solution of 1 M CaCl₂ such that the concentration would be 20 mass% and the gelling bath immersion time was changed to 30 minutes.

The production conditions and results are presented in Table 1 and FIG. 8. FIG. 8 is a photograph showing a polymer aggregate obtained in Example 2 (in which spider silk proteins aggregate to be formed in a fiber form) and a trace drawing which traces the photograph. FIG. 8(A) is a photograph showing a polymer aggregate formed in a gel. FIG. 8(B) is a photograph showing the polymer aggregate from which the gel is removed. FIG. 8(C) is a trace drawing which traces the photograph of FIG. 8(A). FIG. 8(D) is a trace drawing which traces the photograph of FIG. 8(B).

### [Example 3]

A polymer aggregate was produced in a similar manner to Example 1, except that silken thread (not removing sericin) was used instead of the spider silk protein (NRSH1), the concentration of Ca(SCN)₂ contained in the dope was adjusted to 5 M, the gelling bath immersion time was changed to 180 minutes, and the silk was immersed for 60 minutes in RO water (RO water immersion time) after being immersed in the coagulation liquid.

As the silken thread (not removing sericin), silken thread obtained by taking a silkworm pupa out from the cocoon and cutting the cocoon shell was used.

The production conditions and results are presented in Table 1 and FIG. 9. FIG. 9 is a photograph showing a polymer aggregate obtained in Example 3 (in which silk proteins aggregate to be formed in a fiber form) and a trace drawing which traces the photograph. FIG. 9(A) is a photograph showing a polymer aggregate formed in a gel. FIG. 9(B) is a trace drawing which traces the photograph of FIG. 9(A). Incidentally, the polymer aggregate of Example 3 was integrated with the gel.

### [Example 4]

A polymer aggregate was produced in a similar manner to Example 3, except that filamentous fibroin obtained by removing sericin from the silken thread was used instead of the silken thread (not removing sericin), the sodium alginate concentration in the coagulation liquid was changed to 2.0 mass%, the gelling bath immersion time was changed to 30 minutes, and the RO water immersion time was changed to 30 minutes.

The filamentous fibroin obtained by removing sericin from the silken thread was prepared as follows.
(1) Silken thread was put into 0.5 mass% of boiling Marseilles soap water (Marseilles soap was grated by a grater for use) such that the bath ratio would be 1 : 100, and was heated for 30 minutes while stirring occasionally.
(2) The soap water was discarded and the silken thread was rinsed with lukewarm water once.
(3) The silken thread rinsed with lukewarm water in (2) was put in boiling water and heated such that the bath ratio would be 1 : 100.
(4) The hot water was discarded.
(5) The procedures (1) to (4) were repeated two more times (three times in total).
(6) Finally, the silken thread was dehydrated and dried at 60°C or lower.

The production conditions and results are presented in Table 1 and FIG. 10. FIG. 10 is a photograph showing a polymer aggregate obtained in Example 4 (in which silk proteins aggregate to be formed in a fiber form) and a trace drawing which traces the photograph. FIG. 10(A) is a photograph showing a polymer aggregate formed in a gel. FIG. 10(B) is a trace drawing which traces the photograph of FIG. 10(A). Incidentally, the polymer aggregate of Example 4 was integrated with the gel.

### [Example 5]

A polymer aggregate was produced in a similar manner to Example 4, except that regenerated silken thread was used instead of the filamentous fibroin obtained by removing sericin from the silken thread.

The regenerated silken thread was prepared as follows.
(1) Silken thread was heated for 30 minutes in 0.5 mass% of boiling Marseilles soap water (Marseilles soap was grated by a grater for use).
(2) Thereafter, the silken thread was heated for 30 minutes in boiling water.
(3) The procedures (1) and (2) were repeated two more times (three times in total).
(4) Finally, the silken thread was heated for 30 minutes in boiling water.
(5) The silken thread was dried overnight in an environment of a temperature of 37°C.
(6) The silk after drying was weighed, an aqueous solution of 9 M lithium bromide was added thereto in an amount of 10 times the weight of the silk, and the silk was dissolved in an environment of 40°C.
(7) The dissolved solution obtained in (6) was placed in a cellulose dialysis membrane (Seamless Cellulose Tubing manufactured by VISKASESELES COAP, 36/32), and dialyzed with pure water for 3 to 4 days.
(8) The collected solution after dialysis was filtered using filters (hole diameters of 197 µm and 560 µm) to remove dusts.
(9) The protein concentration was measured by a BCA method, and the solution was diluted with MilliQ such that the protein concentration would be 2 mass% or less.
(10) The regenerated silken thread solution was freeze-dried in an environment of -80°C. The thickness was 1 to 2 cm.
(11) After freeze-drying, the solution was stored at 4°C.

The production conditions and results are presented in Table 1. Incidentally, the polymer aggregate of Example 5 was integrated with the gel.

### [Example 6]

A polymer aggregate was produced in a similar manner to Example 1, except that a commercially available gelatin (Catalog Number 170-6537 manufactured by Bio-Rad Laboratories) was used instead of the spider silk protein (NRSH1) and the gelling bath immersion time was changed to 30 minutes.

The production conditions and results are presented in Table 1 and FIG. 11. FIG. 11 is a photograph showing a polymer aggregate obtained in Example 6 (in which gelatin proteins aggregate to be formed in a fiber form) and a trace drawing which traces the photograph. FIG. 11(A) is a photograph showing a polymer aggregate formed in a gel. FIG. 11(B) is a trace drawing which traces the photograph of FIG. 11(A). Incidentally, the polymer aggregate of Example 6 was integrated with the gel.

**[Table 1]**

| | Example 1 | Example 2 | Example 3 | Example 4 | Example 5 | Example 6 |
|---|---|---|---|---|---|---|
| Type of protein | Spider silk (NRSH1) | Spider silk (NRSH1) | Silken thread containing sericin | Silken thread removing sericin | Regenerated silken thread | Gelatin |
| Protein concentration (mass%) | 20 | 20 | 20 | 20 | 20 | 20 |
| Solvent of protein solution | H₂O | DMSO | H₂O | H₂O | H₂O | H₂O |
| Salt contained in dope | Ca(SCN)₂ | CaCl₂ | Ca(SCN)₂ | Ca(SCN)₂ | Ca(SCN)₂ | Ca(SCN)₂ |
| Concentration of salt contained in dope | 1 M | 1M | 5 M | 5 M | 5 M | 1 M |
| Salt contained in coagulation liquid | Na-ALG | Na-ALG | Na-ALG | Na-ALG | Na-ALG | Na-ALG |
| Concentration of salt contained in coagulation liquid (mass%) | 1.0 | 1.0 | 1.0 | 2.0 | 2.0 | 1.0 |
| Gelling bath immersion time (min) | 90 | 30 | 180 | 30 | 30 | 30 |
| RO water immersion time (min) | - | - | 60 | 30 | 30 | - |
| Color of polymer aggregate | White | White | White | White | White | Transparent |
| State of polymer aggregate and gel | Easily separated | Easily separated | Integrated | Integrated | Integrated | Integrated |

As presented in Table 1 and FIGS. 7 to 11, a protein aggregate was obtainable inside the gel without depending on the type of protein. In addition, elution of the protein to the coagulation liquid was almost not confirmed, and an aggregate was obtainable in high yield.

### Reference Signs List

1: polymer solution (dope), 2: extruded dope, 3, 6: container, 4: anion solution (coagulation liquid), 5: gel, 7: water, 8: polymer aggregate, 9a: syringe, 9b: slit hole, 10: schematic cross-sectional view illustrating coagulation liquid-gel-dope interface, 11: dope layer, 12: gel layer, 13: coagulation liquid layer, 21: extruder, 22: dope, 23: nozzle, 24: extruded dope, 25: coagulation bath, 26: coagulation liquid, 27: gel, 28: aggregated fibroin, 29: water bath, 30: pure water, 31: dryer, 32: yarn roll.

## Claims

1. A method for producing a polymer aggregate, comprising:
a step for bringing a polymer solution containing a polymer substance and cations for gel formation into contact with an anion solution containing anions for gel formation to form a gel at an interface between the polymer solution and the anion solution; and
a step for causing the polymer substance to aggregate inside the gel.

2. The method for producing a polymer aggregate according to claim 1, wherein the cations for gel formation are at least one selected from multivalent cations or hydrogen ions.

3. The method for producing a polymer aggregate according to claim 1 or 2, wherein the anions for gel formation are alginate ions.

4. The method for producing a polymer aggregate according to any one of claims 1 to 3, wherein the anion solution further contains an aggregation promoter for the polymer substance.

5. The method for producing a polymer aggregate according to any one of claims 1 to 4, wherein the polymer solution further contains a dissolution promoter for the polymer substance.

6. The method for producing a polymer aggregate according to any one of claims 1 to 5, wherein solvents of the polymer solution and the anion solution are water.

7. The method for producing a polymer aggregate according to any one of claims 1 to 6, wherein the polymer substance is a protein.

8. The method for producing a polymer aggregate according to claim 7, wherein the protein is a polypeptide derived from a spider silk protein.
